# EUROPEAN PATENT APPLICATION

(11) **EP 4 620 958 A1**
(43) Date of publication of application: **24.09.2025**
(21) Application number: 23890787.7
(22) Date of filing: 14.11.2023
(51) Int. Cl.: C07D 487/04, A61K 31/4985, A61P 35/00

(54) **CRYSTAL OF SUBSTITUTED PIPERAZINE DERIVATIVE AND PREPARATION METHOD THEREFOR**

(30) Priority: 14.11.2022 CN 202211417721
(71) Applicant: Kangbaida (Sichuan) Biotechnology Co., Ltd., Chengdu, Sichuan 610000 (CN)
(72) Inventor: LIU, Zhaojun, Chengdu, Sichuan 610000 (CN); CHU, Hongzhu, Chengdu, Sichuan 610000 (CN); ZHU, Yuqin, Chengdu, Sichuan 610000 (CN); CAO, Xin, Chengdu, Sichuan 610000 (CN); WEI, Yonggang, Chengdu, Sichuan 610000 (CN); SUN, Yi, Chengdu, Sichuan 610000 (CN)
(74) Representative: Kador & Partner Part mbB
(86) International application number: PCT/CN2023/131627
(87) International publication number: WO 2024/104354

(57) **Abstract**

Provided are a crystal of substituted piperazine derivative and a preparation method therefor. Specifically, provided are crystalline forms A to I of a compound represented by formula ① and a preparation method therefor.

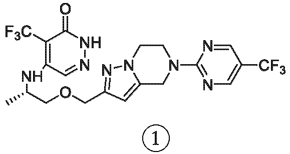

## Description

### TECHNICAL FIELD

The present invention relates to a crystal of substituted piperazine derivative and a preparation method thereof. Specifically, the present invention provides crystalline forms A to I of the compounds represented by formula ① and preparation method thereof.

### BACKGROUND ART

Adenosine diphosphate-ribosylation (ADP-ribosylation) is the post-transcriptional modification process of the proteins, in which single or multiple adenosine diphosphate ribose (ADP-ribose) groups are embedded in amino acid residues of proteins. ADP-ribosylation is a reversible process that involves physiological regulation such as cell signaling, DNA damage repair, transcription, gene expression regulation as well as cell apoptosis. The ADP-ribose is derived from redox cofactor which is nicotinamide adenine dinucleotide (NAD+), and the enzyme that mediates ADP-ribose embedding modification is ADP-ribosylase. In the physiological response regulation, the N-glycosidic bond of NAD+ connecting the ADP-ribose molecule and the nicotinamide group is cleaved, and then it is captured to form a bond with the corresponding amino acid residue of the target protein. ADP-ribosylase can perform two types of modifications: mono-ADP ribosylation and poly-ADP ribosylation. When DNA is damaged or cells are stressed, the PARP is activated, resulting in an increase in the amount of poly ADP-ribose and a decrease in the amount of NAD+. For more than a decade, PARP1 has long been considered the only poly (ADP-ribose) polymerase in mammalian cells and is therefore the most studied enzyme. Scientists have identified 17 different PARPs so far. The MonoPARP occupies majority of PARP family and mediates the important biological functions and various stress responses such as unfolded protein response, NF-κB signaling, antiviral response and cytokine signaling. 2,3,7,8-Tetrachlorodibenzo-p-dioxin (TCDD)-inducible poly(ADP-ribose) polymerase (PARP-7) is a member of MonoPARP family, and its expression is regulated by the aryl hydrocarbon receptor (AHR) activated by TCDD. AHR is a ligand-activated transcription factor that can mediate toxic activities of many environmental xenobiotics. AHR upregulates the expression of PARP-7. PARP-7 interacts with the kinase TBK1 and causes its ADP-ribosylation, leading to the inhibition of TBK1 activity and downregulation of IFN-I (type I interferon) response, thereby inhibiting body's antiviral and tumor immune responses. In terms of PARP-7 inhibitors, no clinical results have been reported so far, so research on PARP-7 inhibitors is of great significance.

PCT/CN2022/094124 discloses a piperazine derivative, whose chemical name is (S)-4-(trifluoromethyl)-5-((1-((5-(5-(5-(trifluoromethyl)pyrimidin-2-yl)-4,5,6,7-tetrahydropyrazolo[1,5-a]pyrazin-2-yl)methoxy)propan-2-yl)amino)pyridazin-3(2H)-one, providing new treatment options for patients.

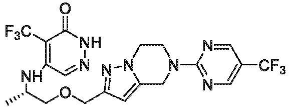

The crystalline form of a pharmaceutically active ingredient often affects the chemical and physical stability of the drug. Different crystalline forms, preparation methods and storage conditions may lead to changes in crystalline form of the compound, and sometimes also be accompanied by the production of other crystalline forms. Amorphous drug products generally do not have a regular crystal structure and often have other defects, such as poor product stability, difficulty in filtration, easy agglomeration, poor fluidity, etc. In view of the importance of crystalline forms as well as stabilities of the solid drugs in the clinical treatment, in-depth research on the crystalline forms of compound (S)-4-(trifluoromethyl)-5-((1-((5-(5-(5-trifluoromethyl)pyrimidin-2-yl)-4,5,6,7-tetrahydropyrazolo[1,5-a]pyrazin-2-yl)methoxy)propan-2-yl)amino)pyridazin-3(2H)-one is of a great significance for the development of drugs suitable for industrial production and with good biological activity.

### SUMMARY

The present invention provides a crystal of the (S)-4-(trifluoromethyl)-5-((1-((5-(5-(5-(trifluoromethyl)pyrimidin-2-yl)-4,5,6,7-tetrahydropyrazolo[1,5-a]pyrazin-2-yl)methoxy)propane-2-yl)amino)pyridazin-3(2H)-one (compound ①). The chemical structure of compound ① is as follows:

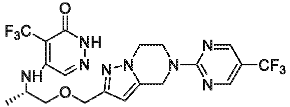

The crystal of the present invention exhibits at least one of following advantages: good solubility, high stability, ease of processing, handling and purifying, improved oral bioavailability of drugs, extended storage period of drug, and ease of manufacturing in various dosage forms.

The crystal of the present invention exhibits pharmaceutical advantages over amorphous forms of compound ①. In particular, the crystal has enhanced chemical and physical stability, which is more conducive to the preparation of solid pharmaceutical dosage forms containing pharmacologically active ingredients.

The crystalline form of present invention is present in about 5% to about 100% by weight of the active pharmaceutical ingredient (API). In some embodiments, the crystalline form of the present invention is present in about 10% to about 100% by weight of the API. In some embodiments, the crystalline form of present invention is present in about 15% to about 100% by weight of the the API. In some embodiments, the crystalline form of the present invention is present in about 20% to about 100% by weight of the API. In some embodiments, the crystalline form of present invention is present in about 25% to about 100% by weight of the API. In some embodiments, the crystalline form of the present invention is present in about 30% to about 100% by weight of the API. In some embodiments, the crystalline form of present invention is present in about 35% to about 100% by weight of the API. In some embodiments, the crystalline form of the present invention is present in about 40% to about 100% by weight of the API. In some embodiments, the crystalline form of the present invention is present in about 45% to about 100% by weight of the API. In some embodiments, the crystalline form of the present invention is present in about 50% to about 100% by weight of the API. In some embodiments, the crystalline form of the present invention is present in about 55% to about 100% by weight of the API. In some embodiments, the crystalline form of the present invention is present in about 60% to about 100% by weight of the API. In some embodiments, the crystalline form of the present invention is present in about 65% to about 100% by weight of the API. In some embodiments, the crystalline form of the present invention is present in about 70% to about 100% by weight of the API. In some embodiments, the crystalline form of the present invention is present in about 75% to about 100% by weight of the API. In some embodiments, the crystalline form of the present invention is present in about 80% to about 100% by weight of the API. In some embodiments, the crystalline form of the present invention is present in about 85% to about 100% by weight of the API. In some embodiments, the crystalline form of the present invention is present in about 90% to about 100% by weight of the API. In some embodiments, the crystalline form of the present invention is present in about 95% to about 100% by weight of the API. In some embodiments, the crystalline form of the present invention is present in about 98% to about 100% by weight of the API. In some embodiments, the crystalline form of the present invention is present in about 99% to about 100% by weight of the aAPI. In some embodiments, substantially all the API is the crystalline form of the present invention, that is, the API is substantially phase-pure crystal.

Unless otherwise specified, compound ① of the present invention is the amorphous form of compound ①.

One embodiment of a crystal of the present invention is crystalline form A of compound ①, wherein X-ray powder diffraction pattern of crystalline form A has characteristic diffraction peaks at the following 2θ positions: 10.389°±0.3°, 11.917°±0.3°, 12.912°±0.3°, 13.385°±0.3°, 14.054°±0.3°, 15.316°±0.3° and 16.636°±0.3°.

Furthermore, the X-ray powder diffraction pattern of crystalline form A has characteristic diffraction peaks at the following 2θ positions: 6.716°±0.3°, 10.043°±0.3°, 10.389°±0.3°, 11.917°±0.3°, 12.912°±0.3°, 13.385°±0.3°, 14.054°±0.3°, 15.316°±0.3°, 16.636°±0.3°, 18.003°±0.3°, 20.014°±0.3°, 20.794°±0.3° and 23.855°±0.3°.

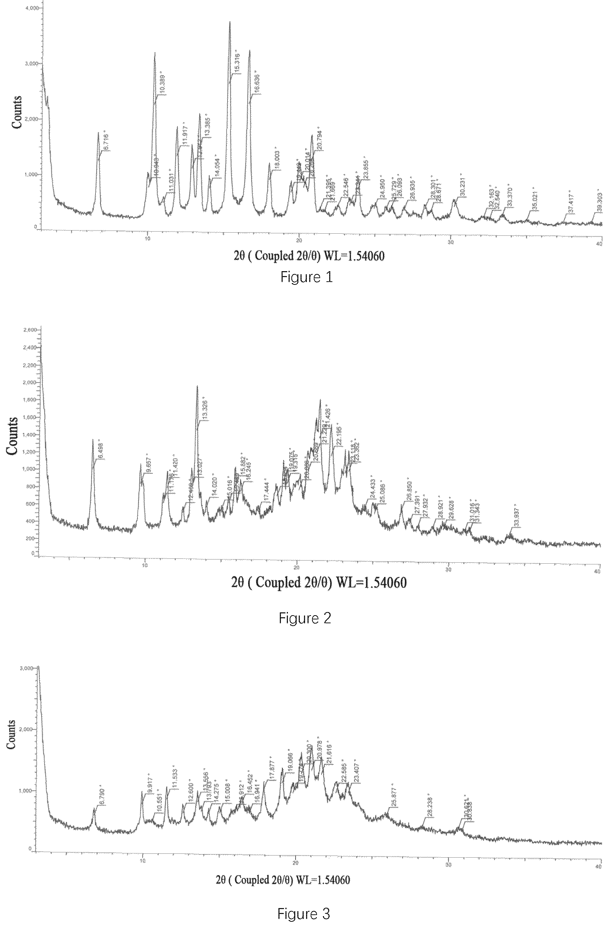

Furthermore, the X-ray powder diffraction pattern of crystalline form A is substantially as shown in FIG. 1.

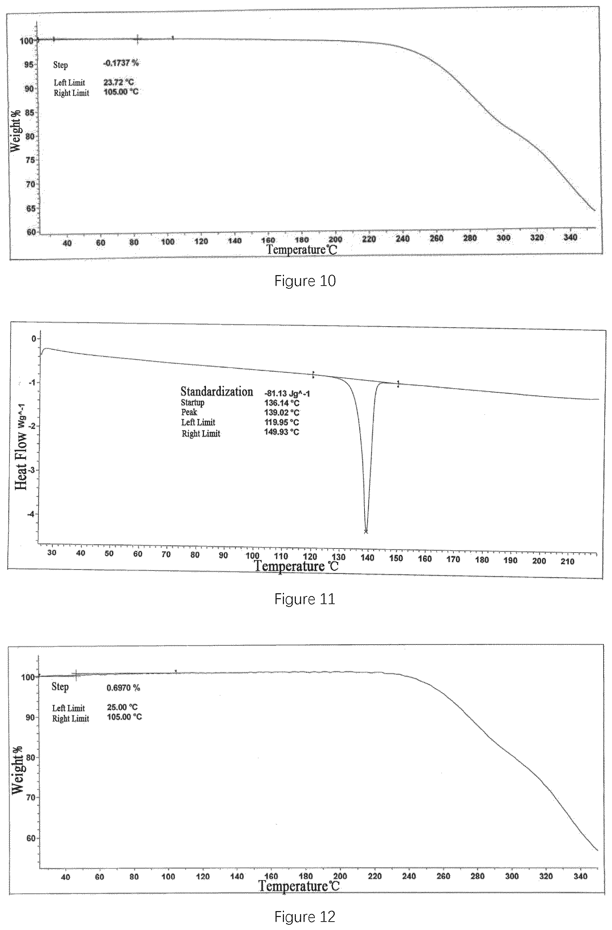

The TGA curve of crystalline form A described in the present invention is substantially as shown in FIG. 10.

The DSC curve of crystalline form A described in the present invention is substantially as shown in FIG. 11.

The present invention also relates to a method for preparing crystalline form A, wherein the compound represented by formula ① is crystallized in solvent (①-A) to obtain crystalline form A, and wherein the solvent (①-A) is selected from any one of acetonitrile, ethanol, n-propanol, acetone and water, or a mixed solvent of any combination of acetonitrile, ethanol, n-propanol, acetone and water in any ratio.

Furthermore, the method for preparing crystalline form A is that the compound represented by formula ① or the crude product thereof is added to solvent (①-A-1), then heated to dissolve the compound or the crude product thereof, then solvent (①-A-2) and solvent (①-A-3) are added, then heated to dissolve it, then cooled and stirred continuously, and then allowed to stand for crystallization to obtain crystalline form A, wherein the solvent (①-A-1), the solvent (①-A-2), and the solvent (①-A-3) are selected from any one of acetonitrile, ethanol, n-propanol, acetone and water.

One embodiment of a crystal of the present invention is crystalline form B of compound ①, wherein X-ray powder diffraction pattern of crystalline form B has characteristic diffraction peaks at the following 2θ positions: 6.498°±0.3°, 13.326°±0.3°, 21.229°±0.3°, 21.426°±0.3° and 22.195°±0.3°.

Furthermore, the X-ray powder diffraction pattern of crystalline form B has characteristic diffraction peaks at the following 2θ positions: 6.498°±0.3°, 9.657°±0.3°, 11.420°±0.3°, 13.027°±0.3°, 13.326°±0.3°, 15.882°±0.3°, 19.075°±0.3°, 19.315°±0.3°, 20.669°±0.3°, 21.229°±0.3°, 21.426°±0.3°, 22.195°±0.3°, 23.118°±0.3° and 23.362°±0.3°.

Furthermore, the X-ray powder diffraction pattern of crystalline form B is substantially as shown in FIG. 2.

The present invention also relates to a method for preparing crystalline form B, wherein the compound represented by formula ① is slurried in the solvent (①-B) to obtain crystalline form B, and wherein solvent (①-B) is selected from n-hexane.

One embodiment of a crystal of the present invention is crystalline form C of compound ①, wherein X-ray powder diffraction pattern of crystalline form C has characteristic diffraction peaks at the following 2θ positions: 19.066°±0.3°, 20.300°±0.3°, 20.978°±0.3° and 21.616°±0.3°.

Furthermore, the X-ray powder diffraction pattern of crystalline form C has characteristic diffraction peaks at the following 2θ positions: 6.790°±0.3°, 9.917°±0.3°, 11.533°±0.3°, 12.600°±0.3°, 13.556°:1:0.3°, 13.793°±0.3°, 14.275°±0.3°, 15.008°±0.3°, 16.452°±0.3°, 17.877°±0.3°, 19.066°±0.3°, 19.774°±0.3°, 20.300°±0.3°, 20.978°±0.3°, 21.616°±0.3°, 22.585°±0.3° and 23.407°±0.3°.

Furthermore, the X-ray powder diffraction pattern of crystalline form C is substantially as shown in FIG. 3.

The present invention also relates to a method for preparing crystalline form C, wherein the compound represented by formula ① is crystallized in solvent (①-C) to obtain crystalline form C, and wherein solvent (①-C) is selected from isopropyl acetate, n-hexane or a mixed solvent of isopropyl acetate and n-hexane.

One embodiment of a crystal of the present invention is crystalline form D of compound ①, wherein X-ray powder diffraction pattern of crystalline form D has characteristic diffraction peaks at the following 2θ positions: 6.646°±0.3° and 13.175°±0.3°.

Furthermore, the X-ray powder diffraction pattern of crystalline form D has characteristic diffraction peaks at the following 2θ positions: 6.646°±0.3°, 13.175°±0.3°, 13.489°±0.3°, 16.450°±0.3°, 20.950°±0.3°, 21.585°±0.3°, 22.369°±0.3°, 23.036°±0.3° and 23.281°±0.3°.

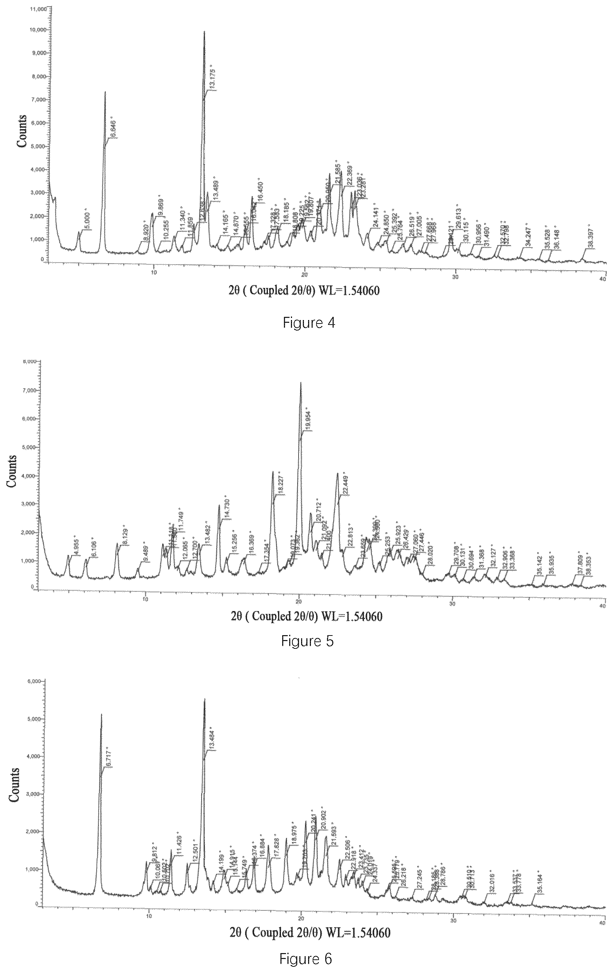

Furthermore, the X-ray powder diffraction pattern of crystalline form D is substantially as shown in FIG. 4.

The present invention also relates to a method for preparing crystalline form D, wherein the compound represented by formula ① is extracted with solvent (①-D), concentrated and dried under vacuum to obtain crystalline form D, and wherein solvent (①-D) is selected from ethyl acetate.

One embodiment of crystal of the present invention is crystalline form E of compound ①, wherein X-ray powder diffraction pattern of crystalline form E has characteristic diffraction peaks at the following 2θ positions: 18.227°±0.3°, 19.954°±0.3° and 22.449°±0.3°.

Furthermore, the X-ray powder diffraction pattern of crystalline form E has characteristic diffraction peaks at the following 2θ positions: 8.129°±0.3°, 11.118°±0.3°, 11.360°±0.3°, 11.749°±0.3°, 13.482°±0.3°, 14.730°±0.3°, 18.227°±0.3°, 19.954°±0.3°, 20.712°±0.3°, 21.092°±0.3°, 22.449°±0.3°, 22.813°±0.3°, 24.399°±0.3°, 24.560°±0.3°, 25.923°±0.3°, 26.429°±0.3°, 27.060°±0.3° and 27.446°±0.3°.

Furthermore, the X-ray powder diffraction pattern of the crystalline form E is substantially as shown in FIG. 5.

The present invention also relates to a method for preparing crystalline form E, wherein the compound represented by formula ① is crystallized in solvent (①-E) to obtain crystalline form E, and wherein the solvent (①-E) is selected from ethyl acetate, n-hexane or a mixed solvent of ethyl acetate and n-hexane.

One embodiment of a crystal of the present invention is crystalline form F of compound ①, wherein X-ray powder diffraction pattern of crystalline form F has characteristic diffraction peaks at the following 2θ positions: 6.717°±0.3° and 13.484°±0.3°.

Furthermore, the X-ray powder diffraction pattern of crystalline form F has characteristic diffraction peaks at the following 2θ positions: 6.717°±0.3°, 9.812°±0.3°, 11.426°±0.3°, 12.501°±0.3°, 13.484°±0.3°, 14.915°±0.3°, 16.374°±0.3°, 16.884°±0.3°, 17.828°±0.3°, 18.975°±0.3°, 20.241°±0.3°, 20.902°±0.3°, 21.593 ±0.3 , 22.506°±0.3°, 22.918°±0.3° and 23.412°±0.3°.

Furthermore, the X-ray powder diffraction pattern of crystalline form F is substantially as shown in FIG. 6.

The TGA curve of crystalline form F described in the present invention is substantially as shown in FIG. 12.

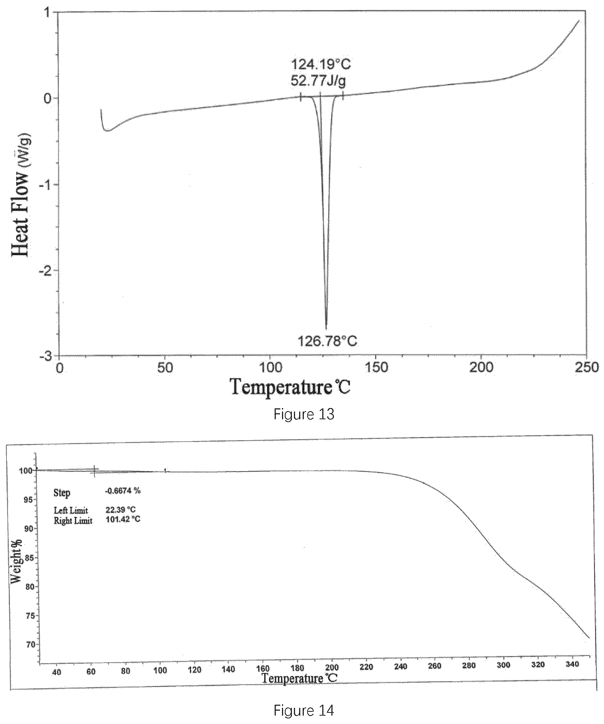

The DSC curve of crystalline form F described in the present invention is substantially as shown in FIG. 13.

The present invention also relates to a method for preparing the crystalline form F, wherein the compound represented by formula ① is crystallized in the solvent (①-F) to obtain the crystalline form F, and wherein the solvent (①-F) is selected from n-propanol, n-heptane or a mixed solvent of n-propanol and n-heptane.

One embodiment of a crystal of the present invention is crystalline form G of compound ①, wherein X-ray powder diffraction pattern of crystalline form G has characteristic diffraction peaks at the following 2θ positions: 6.743°±0.3° and 13.503°±0.3°.

Furthermore, the X-ray powder diffraction pattern of crystalline form G has characteristic diffraction peaks at the following 2θ positions: 6.743°±0.3°, 13.503°±0.3°, 16.901°±0.3°, 18.996°±0.3°, 20.260°±0.3°, 20.920°±0.3° and 21.604±0.3°.

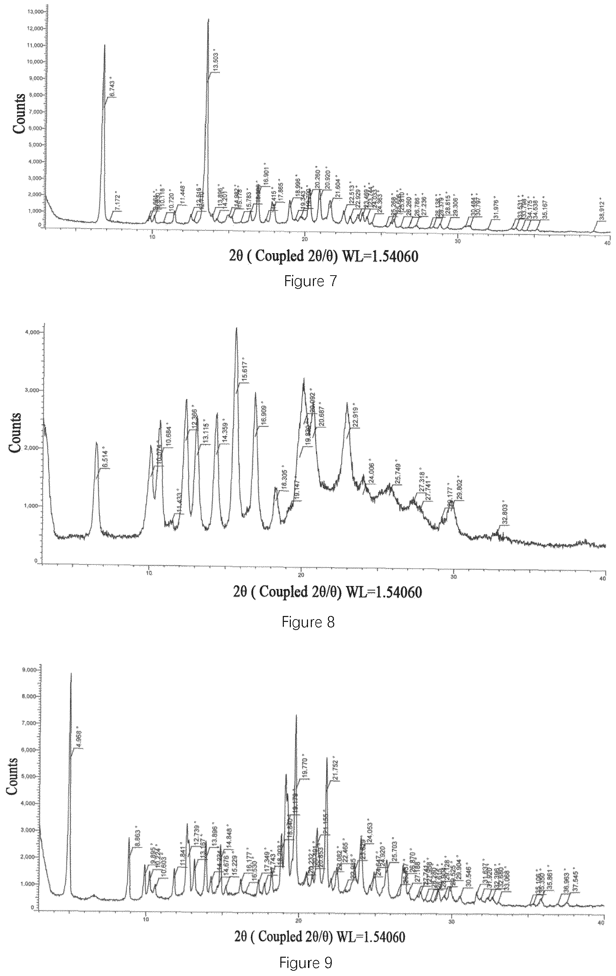

Furthermore, the X-ray powder diffraction pattern of crystalline form G is substantially as shown in FIG. 7.

The present invention also relates to a method for preparing crystalline form G, wherein the compound represented by formula ① is crystallized in the solvent (①-G) to obtain crystalline form G, and wherein the solvent (①-G) is selected from ethanol, n-heptane or mixed solvent of ethanol and n-heptane.

One embodiment of a crystal of the present invention is crystalline form H of compound ①, wherein X-ray powder diffraction pattern of crystalline form H has characteristic diffraction peaks at the following 2θ positions: 12.366°±0.3°, 13.115°±0.3°, 14.359°±0.3°, 15.617°±0.3° and 16.909°±0.3°.

Furthermore, the X-ray powder diffraction pattern of crystalline form H has characteristic diffraction peaks at the following 2θ positions: 6.514°±0.3°, 10.074°±0.3°, 10.684°±0.3°, 12.366°±0.3°, 13.115°±0.3°, 14.359°±0.3°, 15.617°±0.3°, 16.909°±0.3°, 19.839°±0.3°, 20.092°±0.3°, 20.687°±0.3° and 22.919°±0.3°.

Furthermore, the X-ray powder diffraction pattern of crystalline form H is substantially as shown in FIG. 8.

The TGA curve of crystalline form H described in the present invention is substantially as shown in FIG. 14.

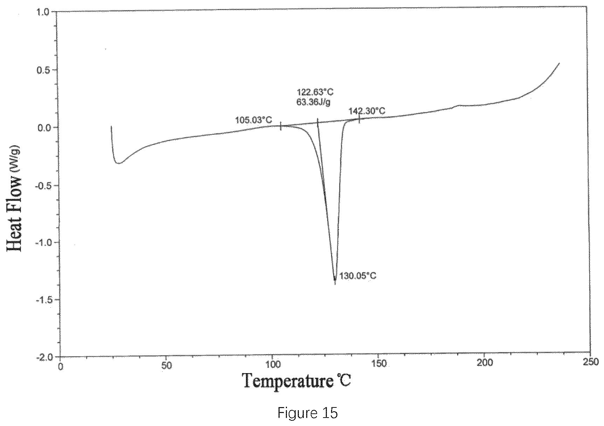

The DSC curve of crystalline form H described in the present invention is substantially as shown in FIG. 15.

The present invention also relates to a method for preparing crystalline form H, wherein the compound represented by formula ① is crystallized in solvent (①-H) to obtain the crystalline form H, and wherein the solvent (①-H) is selected from any one of acetonitrile, ethanol, n-propanol, acetone and water, or a mixed solvent of any combination of acetonitrile, ethanol, n-propanol, acetone and water in any ratio.

One embodiment of a crystal of the present invention is crystalline form I of compound ①, wherein X-ray powder diffraction pattern of crystalline form I has characteristic diffraction peaks at the following 2θ positions: 4.968°±0.3°, 19.770°±0.3° and 21.752°±0.3°.

Furthermore, the X-ray powder diffraction pattern of crystalline form I has characteristic diffraction peaks at the following 2θ positions: 4.968°±0.3°, 8.863°±0.3°, 12.739°±0.3°, 13.896°±0.3°, 14.848°±0.3°, 18.840°±0.3°, 19.179°±0.3°, 19.770°±0.3°, 21.155°±0.3°, 21.752°±0.3° and 24.053°±0.3°.

Furthermore, the X-ray powder diffraction pattern of crystalline form I is substantially as shown in FIG. 8.

The present invention also relates to a method for preparing crystalline form I, wherein the compound represented by formula ① is crystallized in solvent (①-I) to obtain crystalline form I, and wherein the solvent (①-I) is selected from acetonitrile, water or a mixed solvent of acetonitrile and water.

The present invention also relates to a pharmaceutical composition, which comprises the therapeutically effective amount of the crystalline compound described in the present invention and one or more pharmaceutically accepted carriers or excipients.

The crystals of the present invention can be used as active pharmaceutical ingredients, or pharmaceutical compositions in which the crystals are used as active ingredients, can be used for treating and/or preventing cancer.

In one or more embodiments, the present invention provides use of the crystals of the present invention as active pharmaceutical ingredients, or use of pharmaceutical compositions in which the crystals are used as active ingredients for treating and/or preventing solid tumors.

In one or more embodiments, the solid tumor of the present invention is selected from non-small cell lung cancer, head and neck squamous cell carcinoma, esophageal squamous cell carcinoma, hormone receptor positive (HR+) breast cancer, colon cancer or PARP7 amplified advanced solid tumor.

The X-ray powder diffraction pattern substantially identical to those disclosed in the present invention shall also belong to the scope of the present invention.

It is understood that the melting peak height of the DSC curve depends on many factors related to sample preparation and instrument geometry, as is well known in the field of differential scanning calorimetry (DSC), while the peak position is relatively insensitive to experimental details. Therefore, in some embodiments, the crystalline compound of the present invention is characterized by DSC pattern with characteristic peak positions, having substantially the same properties as the DSC pattern provided in accompanying drawings of the present invention, with an error tolerance of ± 3 °C.

Unless stated to the contrary, the terms used in description and claims have the following meanings:
The term "effective dose" refers to the amount of the compound that causes physiological or medical translation of a tissue, system or subject that is being sought, including an amount of the compound that is sufficient to prevent the occurrence of one or more symptoms of the disorder or disease being treated or to alleviate them to some extent when administered to the subject.

"IC₅₀" refers to the half inhibitory concentration, which is the concentration at which half of the maximum inhibitory effect is achieved.

The crystalline forms of the present invention can be analyzed by using various analytical techniques known to those ordinary skilled in the art, including but are not limited to X-ray powder diffraction (XRD).

It is understood that the numerical values described and protected by the present invention are approximate values. The variation in numerical values may be due to calibration of equipment, equipment error, purity of the crystal, crystal size, sample size and other factors.

It can be understood that the crystals of the present invention are not limited to having the same characteristic patterns, such as XRD, as described in drawings disclosed in present invention. Any crystalline forms having characteristic pattern that is substantially the same or essentially the same as the patterns described in the drawings falls within the scope of the present invention.

Without departing from the scope and spirit of the present invention, various modification and alteration of the present invention will be apparent to those skilled in the art upon consideration of the disclosure of the specification and procedures in the examples of the present invention.

Unless otherwise stated, abbreviations used in description and claims have the following meanings:

| **Abbreviation** | **Technical Term** |
|---|---|
| XRPD | X-ray powder diffraction |
| TGA | Thermogravimetric analysis |
| DSC | Differential scanning calorimetry |

### DESCRIPTION OF THE DRAWINGS

FIG. 1 is an X-ray powder diffraction patternof crystalline form A.
FIG. 2 is an X-ray powder diffraction pattern of crystalline form B.
FIG. 3 is an X-ray powder diffraction pattern of crystalline form C.
FIG. 4 is an X-ray powder diffraction pattern of crystalline form D.
FIG. 5 is an X-ray powder diffraction pattern of crystalline form E.
FIG. 6 is an X-ray powder diffraction pattern of crystalline form F.
FIG. 7 is an X-ray powder diffraction pattern of crystalline form G.
FIG. 8 is an X-ray powder diffraction pattern of crystalline form H.
FIG. 9 is an X-ray powder diffraction pattern of crystalline form I.
FIG. 10 is a TGA thermogram of crystalline form A.
FIG. 11 is a DSC thermogram of crystalline form A.
FIG. 12 is a TGA thermogram of crystalline form F.
FIG. 13 is a DSC thermogram of crystalline form F.
FIG. 14 is a TGA thermogram of crystalline form H.
FIG. 15 is a DSC thermogram of crystalline form H.

### DETAILED DESCRIPTION

The implementation process of the present invention and beneficial effects produced are described in detail below through specific embodiments, which is intended to help readers better understand the essence and characteristics of the present invention, not intended to limit the scope of the implementation of the present invention.

If there is no special explanation in embodiments, the solution refers to aqueous solution.

Unless otherwise specified, experimental conditions for crystallization are generally room temperature (20-30 °C, 30-70% RH), the solvent ratio refers to the volume ratio.

### Intermediate 1

### 5-Chloro-2-(4-methoxybenzyl)-4-(trifluoromethyl)pyridazin-3(2H)-one (Intermediate 1)

### 5-chloro-2-(4-methoxybenzyl)-4-(trifluoromethyl)pyridazin-3(2H)-one

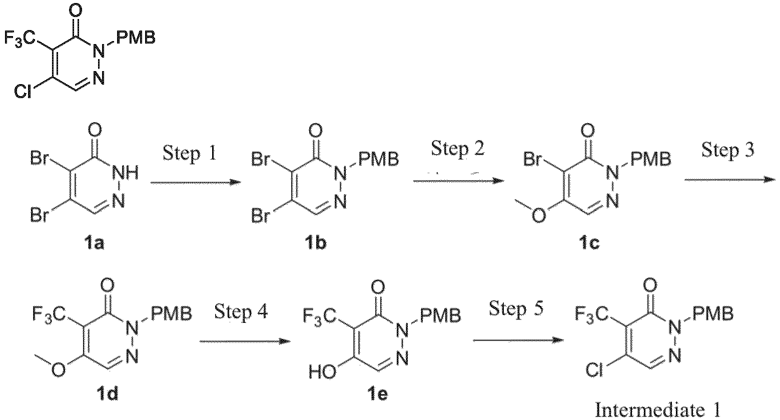

### Step 1:

### 4,5-Dibromo-2-(4-methoxybenzyl)pyridazin-3(2H)-one(1b)

### 4,5-dibromo-2-(4-methoxybenzyl)pyridazin-3(2H)-one

At 0 °C to 10 °C, sodium hydride (11.82 g, 295.41 mmol, 1.5 equiv, 60%) was added in batches to the solution of 4,5-dibromo-2,3-dihydropyridazin-3-one (**1a**, 50 g, 196.94 mmol, 1.0 equiv) in N,N-dimethylformamide (500 mL), then 1-(chloromethyl)-4-methoxybenzene (46.06 g, 294.11 mmol, 1.49 equiv) was added at 0 °C. After the addition was completed, the reaction mixture was stirred at room temperature for 3 hours. After the reaction was completed, the reaction solution was slowly poured into 1.0 L of ice-water mixture for quenching, and was extracted with dichloromethane (2 × 500 mL). Organic layers were combined and concentrated. The solid was washed with methanol (500 mL × 2) to obtain **1b** as a yellow solid (48.4 g, yield 66%).

LC-MS m/z (ESI) = 375.00 [M+1].

### Step 2:

### 4-Bromo-5-methoxy-2-(4-methoxybenzyl)pyridazin-3(2H)-one (1c)

### 4-bromo-5-methoxy-2-(4-methoxybenzyl)pyridazin-3(2H)-one

**1b** (48.4 g, 129.40 mmol, 1.0 equiv) and potassium hydroxide (21.78 g, 388.30 mmol, 3.00 equiv) were dissolved in methanol (417 mL) and the reaction solution was stirred at room temperature for 2 hours. The obtained reaction mixture was concentrated to 80 mL and filtered to obtain the crude product. The obtained filter cake was slurried in water (160 mL) for 1 hour and filtered to obtain **1c** as a white solid (38.72 g, yield 92%).

LC-MS m/z (ESI) = 326.30 [M+1].

### Step 3:

### 5-Methoxy-2-(4-methoxybenzyl)-4-( trifluoromethyl)pyridazin-3(2H)-one (1d)

### 5-methoxy-2-(4-methoxybenzyl)-4-( trifluoromethyl)pyridazin-3(2H)-one

**1c** (14 g, 43.04 mmol, 1.0 equiv) and CuI (4.10 g, 21.52 mmol, 0.50 equiv) were weighed into a 250 mL reaction flask, dissolved in N-methylpyrrolidone (72 mL), and then methyl 2,2-difluoro-2-(fluorosulfonyl) acetate (16.4 mL, 129.11 mmol, 3.0 equiv) was slowly added. After the addition was completed, the reaction was placed in oil bath at 100 °C and stirred for 3 hours. After the reaction was completed, 90 mL of water was added to the reaction solution for quenching. The obtained solution was extracted with dichloromethane (3×60 mL). The organic layers were combined, dried over anhydrous sodium sulfate, and concentrated under vacuum. The residue was purified by the column chromatography (petroleum ether: ethyl acetate = 1:1) to obtain **1d** as a white solid (12.1 g, yield 89%).

LC-MS m/z (ESI) = 315.10 [M+1].

### Step 4:

### 5-Hydroxy-2-(4-methoxybenzyl)-4-(trifluoromethyl)pyridazin-3(2H)-one (1e)

### 5-hydroxy-2-(4-methoxybenzyl)-4-(trifluoromethyl)pyridazin-3(2H)-one

At room temperature, trimethylsilyl iodide (9.97 g, 50.07 mmol, 1.3 equiv) was added dropwise into **1d** (12.1 g, 38.52 mmol, 1.0 equiv) in N, N-dimethylformamide (60 mL). The obtained reaction solution was stirred at 85 °C for 20 hours. After the reaction was completed, 60 mL of water was added to the reaction mixture to quench the reaction, and then the obtained solution was extracted with dichloromethane (3×60 mL). Organic phases were combined, dried over anhydrous sodium sulfate, and concentrated under vacuum. The residue was purified by column chromatography (petroleum ether: ethyl acetate = 1:1) to obtain **1e** as a white solid (10.4 g, yield 90%).

LC-MS m/z (ESI) = 301.07 [M+1].

### Step 5:

### 5-Chloro-2-(4-methoxybenzyl)-4-(trifluoromethyl)pyridazin-3(2H)-one (Intermediate 1)

### 5-chloro-2-(4-methoxybenzyl)-4-(trifluoromethyl)pyridazin-3(2H)-one

At 0 °C, oxalyl chloride (8.79 g, 69.32 mmol, 2.0 equiv) was slowly added dropwise into the solution of compound **1e** (10.4 g, 34.66 mmol, 1.0 equiv) in N,N-dimethylformamide (52 mL) . After the addition was completed, the reaction mixture was stirred at room temperature for 8 hours. After the reaction was completed, 550 mL of water was added to the reaction solution for quenching. The mixture was filtered to obtain **intermediate 1** as a white solid (11.04 g, 99%).

LC-MS m/z (ESI) = 319.68 [M+1].

### Intermediate 2

### (S)-1-((5-(4-methoxybenzyl)-4,5,6,7-tetrahydropyrazolo[1,5-a]pyrazin-2-yl)methoxy)propan-2-amine (Intermediate 2)

### (S)-1-((5-(4-methoxybenzyl)-4,5,6,7-tetrahydropyrazolo[1,5-a]pyrazin-2-yl)methoxy)propan-2-amine

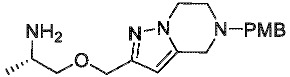

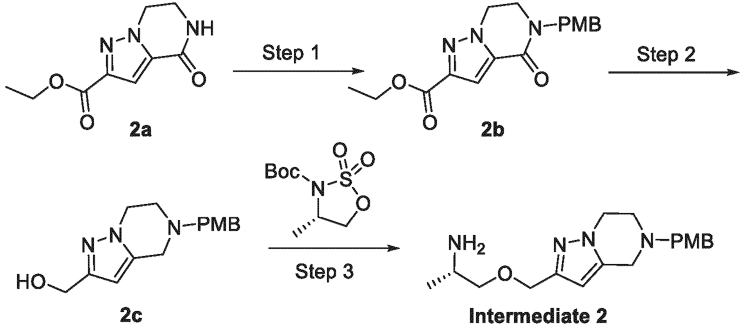

### Step 1:

### Ethyl 5-(4-methoxybenzyl)-4-oxo-4,5,6,7-tetrahydropyrazolo[1,5-a]pyrazine-2-carboxylate (2b)

### ethyl-5-(4-methoxybenzyl)-4-oxo-4,5,6,7-tetrahydropyrazolo[1,5-a]pyrazine-2-carboxylate.

Ethyl 4-oxo-4,5,6,7-tetrahydropyrazolo[1,5-a]pyrazine-2-carboxylate (2a, 5.0 g, 24 mmol, 1.0 equiv) and p-methoxybenzyl bromide (4.2 mL, 28.8 mmol, 1.2 equiv) were weighed out and dissolved in N,N-dimethylformamide (50 mL),. Sodium hydride (1.15 g, 28.8 mmol, 1.2 equiv) was added slowly in an ice bath. After the addition was completed, the reaction was placed at room temperature for 3 hours. After the reaction was completed, water was added for quenching, ethyl acetate was added for extraction, the product was dried with anhydrous sodium sulfate, and then the organic phase was spin dried. The crude product was purified by flash column chromatography (dichloromethane: methanol = 20:1) to give **2b** as a white solid (7.5 g, yield 92%).
¹ H NMR (400 MHz, DMSO-d6): δ 7.33-7.23 (m, 2H), 7.14 (s, 1H), 6.94-6.87 (m, 2H), 4.62 (s, 2H), 4.50-4.38 (m, 2H), 4.28 (q, 2H), 3.76-3.69 (m, 5H), 1.29 (t, 3H)
LC-MS m/z (ESI) = 330.10 [M+1].

### Step 2:

### (5-(4-methoxybenzyl)-4,5,6,7-tetrahydropyrazolo[1,5-a]pyrazin-2-yl)methanol (2c)

### (5-(4-methoxybenzyl)-4,5,6,7-tetrahydropyrazolo[1,5-a]pyrazin-2-yl)methanol

**2b** (4.1 g, 12.5 mmol, 1.0 equiv) was weighed and dissolved in tetrahydrofuran (100 mL). Under nitrogen protection, tetrahydrofuran solution of lithium aluminum tetrahydride (50 mL, 50 mmol, 4.0 equiv) was dropwise added slowly in an ice bath. After the addition was completed, the temperature was increased to 70 °C and the reaction mixture was reacted for 10 minutes. After the reaction was completed, the reaction mixture was cooled to room temperature, the reaction was quenched in an ice-water bath, filtered, the filtrate was spin dried, the crude product was purified by flash column chromatography (dichloromethane: methanol = 10: 1) to obtain **2c** as a yellow solid (2.45 g, yield 71%).

LC-MS m/z (ESI) = 274.10 [M+1].

### Step 3:

### (S)-1-((5-(4-methoxybenzyl)-4,5,6,7-tetrahydropyrazolo[1,5-a]pyrazin-2-yl)methoxy)propan-2-amine (Intermediate 2)

### (S)-1-((5-(4-methoxybenzyl)-4,5,6,7-tetrahydropyrazolo[1,5-a]pyrazin-2-yl)methoxy)propan-2-amine

**2c** (864 mg, 3.16 mmol, 1.0 equiv) was added to a 25 mL reaction flask and dissolved in anhydrous *N*,*N*-dimethylformamide (18 mL). Under N₂ protection, sodium hydride (300 mg, 7.51 mmol, 2.5 eq) was added in batches at 0 °C. After the addition was completed, the reaction mixture was stirred at the temperature for 30 minutes. Subsequently, *tert*-butyl (*S*)-4-methyl-1,2,3-oxathiazolidine-3-carboxylate 2,2-dioxide in *N,N*-dimethylformamide (18 mL) was dropwise added slowly to the reaction system. The temperature was kept at 0 °C during the addition and the reaction system was stirring continuously for 2 hours. After the reaction was completed, the pH value of the reaction system was adjusted to 3 and the reaction system was stirred at room temperature for 0.5 hours. The reaction mixture was extracted with EA (3×120 mL). Organic layers were combined, dried over anhydrous sodium sulfate, and then concentrated under vacuum to obtain a crude product, which was purified by column chromatography (dichloromethane: methanol = 40:1) to obtain **intermediate 2** as a white solid (252 mg, yield 24%). LC-MS m/z (ESI) = 331.50 [M+1].

### Intermediate 3

### (S)-5-((1-((4,5,6,7-tetrahydropyrazolo[1,5-a]pyrazin-2-yl)methoxy)propan-2-yl)amino)-4-(trifluoromethyl)pyridazin-3(2H)-one (Intermediate 3)

### (S)-5-((1-((4,5,6,7-tetrahydropyrazolo[1,5-a]pyrazin-2-yl)methoxy)propan-2-yl)amino)-4-(trifluoromethyl)pyridazin-3(2H)-one

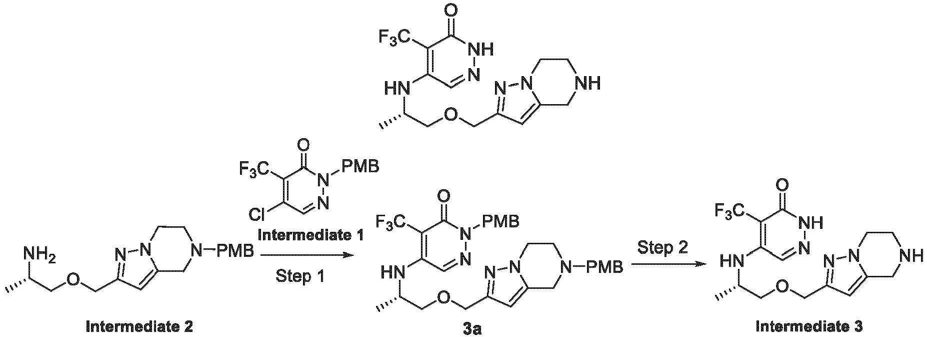

### Step 1:

### (S)-2-(4-methoxybenzyl)-5-((1-((5-(4-methoxybenzyl)-4,5,6,7-tetrahydropyrazolo[1,5-a]pyrazin-2-yl)methoxy)propan-2-yl)amino)-4-(trifluoromethyl)pyridazin-3(2H)-one (3a)

### (S)-2-(4-methoxybenzyl)-5-((1-((5-(4-methoxybenzyl)-4,5,6,7-tetrahydropyrazolo[1,5-a]pyrazin-2-yl)methoxy)propan-2-yl)amino)-4-(trifluoromethyl)pyridazin-3(2H)-one.

**Intermediate 2** (252 mg, 0.764 mmol, 1.0 eq) and **intermediate 1** (291.4 mg, 0.916 mmol, 1.1 equiv) were weighed and placed in a 10 mL reaction flask and *N,N*-dimethylformamide (3.0 mL) was added to dissolve it. Subsequently, *N,N*-diisopropylethylamine (0.5 mL, 3.06 mmol, 4.0 equiv) was added. The mixture was stirred at 100 °C for 4 hours. After the reaction was completed, the product was concentrated under vacuum and the residue was purified by column chromatography (petroleum ether:ethyl acetate = 1:1.5) to obtain **3a** as a white solid (359.8 mg, yield 77%).

LC-MS m/z (ESI) = 613.62 [M+1].

### Step 2:

### (S)-5-((1-(((4,5,6,7-tetrahydropyrazolo[1,5-a]pyrazin-2-yl)methoxy)propan-2-yl)amino)-4-(trifluoromethyl)pyridazin-3(2H)-one (Intermediate 3)

### (S)-5-((1-((4,5,6,7-tetrahydropyrazolo[1,5-a]pyrazin-2-yl)methoxy)propan-2-yl)amino)-4-(trifluoromethyl)pyridazin-3(2H)-one

Trifluoroacetic acid (3.4 mL) and trifluoromethanesulfonic acid (0.42 mL, 4.7 mmol, 8.0 equiv) were added into a 10 mL reaction flask containing **3a** (359.8 mg, 0.588 mmol, 1.0 equiv). After the addition was completed, the reaction was stirred at 25 °C for 1 hour. Subsequently, the reaction solution was stirred in an oil bath at 70 °C. After the reaction was completed, 15 mL of water was added to the reaction solution for quenching. The obtained solution was extracted with ethyl acetate (3×15 mL). The pH value of the organic layer was adjusted to 8 to 9 by potassium carbonate aqueous solution. The organic layers were combined and concentrated under vacuum. The residue was purified by MPLC (water: acetonitrile = 1:1) to obtain **intermediate 3** as a white solid (48 mg, yield 22%).

¹ H NMR (400 MHz, DMSO-d6) δ 12.46 (s, 1H), 8.81 (s, 2H), 7.90 (s, 1H), 6.28 (dd, 1H), 6.11 (s, 1H), 5.01 (s, 2H), 4.41 (d, 2H), 4.32 (t, 2H), 4.16 (t, 3H), 3.55-3.45 (m, 2H), 1.15 (d, 3H).

LC-MS m/z (ESI) = 373.1 [M+1].

### Example 1: Preparation of compound ①

### (S)-4-(Trifluoromethyl)-5-((1-((5-(5-(trifluoromethyl)pyrimidin-2-yl)-4,5,6,7-tetrahydropyrazolo[1,5-a]pyrazin-2-yl)methoxy)propan-2-yl)amino)pyridazin-3(2H)-one (Compound ①)

### (S)-4-(trifluoromethyl)-5-((1-((5-(5-(trifluoromethyl)pyrimidin-2-yl)-4,5,6,7-tetrahydropyrazolo[1,5-a]pyrazin-2-yl)methoxy)propan-2-yl)amino)pyridazin-3(2H)-one

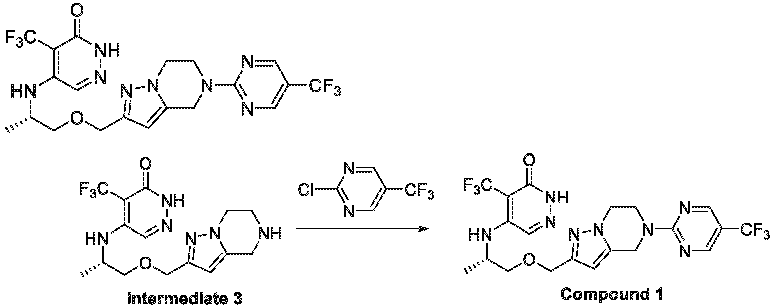

**Intermediate 3** (48 mg, 0.13 mmol, 1.0 equiv) and 2-chloro-5-trifluoromethylpyrimidine (24 mg, 0.13 mmol, 1.0 equiv) were weighed and placed into a 10 mL reaction flask and dissolved in *N,N*-dimethylformamide (4.0 mL). *N,N*-diisopropylethylamine (0.086 mL, 0.52 mmol, 4 equiv) was added to the mixture and the reaction was stirred at 90 °C for 1 hour. After the reaction was completed, the reaction mixture was concentrated under vacuum. The residue was purified by MPLC (water: acetonitrile = 1:1) to obtain **compound ①** as a white solid (40 mg, yield 59%).

¹H NMR (400 MHz, DMSO-d6): δ 12.46 (s, 1H), 8.81 (s, 2H), 7.90 (s, 1H), 6.28 (dd, 1H), 6.11 (s, 1H), 5.01 (s, 2H), 4.41 (d, 2H), 4.32 (t, 2H), 4.16 (t, 3H), 3.55-3.45 (m, 2H), 1.15 (d, 3H).

LCMS m/z = 519.40 [M+1].

### Example 2: Preparation of crystalline form A of compound ①

### Method 1:

Compound ① (7.8 g) was dissolved in acetonitrile (16 mL) at room temperature by stirring, and then water (24 mL) was added. Crystallization was carried out under stirring at room temperature for 4 hours. The obtained solution was filtered and the filter cake was vacuum-dried at 55 °C to obtain crystalline form A.

### Method 2:

Under nitrogen protection, the crude compound ① (5.20 g) was added into acetonitrile (16.50 g), and the temperature was raised to 60 °C to 65 °C for dissolution for 0.5 hours. Water (52.43 g) and n-propanol (8.40 g) were added in sequence, and the temperature was raised to 65±5 °C for dissolution for 0.5 hours. The temperature was lowered to 30±5 °C and stirred continuously for 11 hours. The mixture was allowed to stand for 16 hours for crystallization, and the temperature was maintained at 25±5 °C for further crystallization for 6 hours. The mixture was filtered, washed with water (6.09 g), and dried at 55±5 °C for 16 hours to obtain crystalline form A.

### X-ray powder diffraction analysis:

Crystalline form A of compound ① was characterized by X-ray diffractometer (Bruker D8 advance) using graphite-monochromated Cu Kα radiation (λ = 1.54) at room temperature. Method parameters are as follows: current voltage: -40 mA, 40 Kv; step length: 0.02°; scanning rate: 0.2 sec/step; scanning range: 3°- 40°2θ. The powder diffraction pattern was obtained, and data were analyzed. The X-ray powder diffraction data of crystalline form A are shown in Table 1.

**Table 1: X-ray powder diffraction data of crystalline form A**

| Peak No. | 2θ Angle (°) | Interplanar Spacing | Peak Height | Half Peak Width | Relative Peak Height (%) |
|---|---|---|---|---|---|
| 1 | 6.716 | 13.15137 | 971.810 | 1269.07 | 42.0 |
| 2 | 10.043 | 8.80085 | 526.529 | 796.125 | 22.8 |
| 3 | 10.389 | 8.50788 | 2009.58 | 2287.76 | 86.9 |
| 4 | 11.031 | 8.01415 | 234.582 | 524.733 | 10.1 |
| 5 | 11.917 | 7.42058 | 1060.73 | 1376.04 | 45.9 |
| 6 | 12.912 | 6.85086 | 801.864 | 1146.11 | 34.7 |
| 7 | 13.385 | 6.60991 | 1178.69 | 1532.42 | 51.0 |
| 8 | 14.054 | 6.29663 | 438.239 | 800.657 | 18.9 |
| 9 | 15.316 | 5.78038 | 2312.95 | 2684.47 | 100.0 |
| 10 | 16.636 | 5.32472 | 1950.19 | 2317.13 | 84.3 |
| 11 | 18.003 | 4.92319 | 613.197 | 957.360 | 26.5 |
| 12 | 19.442 | 4.56191 | 407.384 | 740.181 | 17.6 |
| 13 | 20.014 | 4.43280 | 596.936 | 935.388 | 25.8 |
| 14 | 20.266 | 4.37846 | 485.880 | 825.544 | 21.0 |
| 15 | 20.794 | 4.26832 | 980.436 | 1320.10 | 42.4 |
| 16 | 21.395 | 4.14976 | 114.583 | 450.057 | 5.0 |
| 17 | 21.669 | 4.09796 | 65.3330 | 397.429 | 2.8 |
| 18 | 22.546 | 3.94054 | 147.695 | 478.010 | 6.4 |
| 19 | 23.341 | 3.80810 | 193.412 | 525.366 | 8.4 |
| 20 | 23.855 | 3.72713 | 473.983 | 802.844 | 20.5 |
| 21 | 24.950 | 3.56597 | 137.456 | 460.036 | 5.9 |
| 22 | 25.729 | 3.45974 | 111.060 | 426.429 | 4.8 |
| 23 | 26.093 | 3.41236 | 175.423 | 484.866 | 7.6 |
| 24 | 26.935 | 3.30750 | 135.387 | 440.202 | 5.9 |
| 25 | 28.301 | 3.15091 | 172.468 | 469.672 | 7.5 |
| 26 | 28.671 | 3.11106 | 112.206 | 403.383 | 4.9 |
| 27 | 30.231 | 2.95397 | 236.105 | 507.981 | 10.2 |
| 28 | 32.163 | 2.78084 | 65.7828 | 311.226 | 2.8 |
| 29 | 32.540 | 2.74942 | 34.6202 | 279.562 | 1.5 |
| 30 | 33.370 | 2.68297 | 92.9555 | 330.622 | 4.0 |
| 31 | 35.021 | 2.56012 | 52.5080 | 273.920 | 2.3 |
| 32 | 37.417 | 2.40150 | 26.3253 | 29.153 | 1.1 |
| 33 | 39.303 | 2.29050 | 48.2425 | 253.239 | 2.1 |

The X-ray powder diffraction pattern of crystalline form A of compound ① is shown in FIG. 1.

### TGA and DSC analysis:

Crystalline form A of compound ① was subjected to thermogravimetric analysis (TGA) and differential scanning calorimetry (DSC) analysis respectively. TGA and DSC were collected on Mettler thermogravimetric analyzer (Mettler TGA/DSC 3+) and Mettler differential scanning calorimeter (Mettler DSC 3) respectively. The TGA and DSC testing parameters are listed in Table 2. The test results are shown in FIG. 10 and 11 respectively. The TGA thermogram shows that weight loss is 0.1737% when heated to 105 °C. The DSC thermogram shows that peak value of endothermic peak is 139.02 °C.

**Table 2: TGA and DSC testing parameters**

| Parameter | TGA | DSC |
|---|---|---|
| Method | Linear temperature rise | Linear temperature rise |
| Sample pan | Aluminum pan, open | Aluminum pan, gland |
| Temperature range | Room temperature to 350 °C | 25 °C to 300 °C |
| Heating rate | 10 °C /min | 10 °C /min |
| Protective gas | Nitrogen | Nitrogen |

### Example 3: Preparation of crystalline form B of compound ①

Compound ① (10 g) prepared by the reversed-phase purification was dissolved in n-hexane (100 mL), slurried at room temperature for 2 h, filtered, and the obtained solid was vacuum-dried at 55 °C to obtain crystalline form B.

### X-ray powder diffraction analysis:

Crystalline form B of compound ① was characterized by X-ray diffractometer (Bruker D8 advance) using graphite-monochromated Cu Kα radiation (λ = 1.54) at room temperature. Method parameters are as follows: current voltage: -40 mA, 40 Kv; step length: 0.02°; scanning rate: 0.2 sec/step; scanning range: 3°- 40°2θ. The powder diffraction pattern was obtained, and data were analyzed. The X-ray powder diffraction data of crystalline form B are shown in Table

**Table 3: X-ray powder diffraction data of crystalline form B**

| Peak No. | 2θ Angle (°) | Interplanar Spacing | Peak Height | Half Peak Width | Relative Peak Height (%) |
|---|---|---|---|---|---|
| 1 | 6.498 | 13.59189 | 689.350 | 1013.65 | 65.6 |
| 2 | 9.657 | 9.15101 | 523.132 | 857.262 | 49.8 |
| 3 | 11.176 | 7.91080 | 256.917 | 633.967 | 24.4 |
| 4 | 11.420 | 7.74228 | 429.831 | 812.442 | 40.9 |
| 5 | 12.469 | 7.09333 | 121.776 | 527.769 | 11.6 |
| 6 | 13.027 | 6.79053 | 399.069 | 821.224 | 38.0 |
| 7 | 13.326 | 6.63906 | 1051.05 | 1480.58 | 100.0 |
| 8 | 14.020 | 6.31153 | 153.152 | 596.469 | 14.6 |
| 9 | 15.016 | 5.89508 | 103.978 | 567.504 | 9.9 |
| 10 | 14.489 | 5.71637 | 171.491 | 645.671 | 16.3 |
| 11 | 15.882 | 5.57569 | 360.702 | 842.080 | 34.3 |
| 12 | 16.245 | 5.45186 | 289.822 | 776.495 | 27.6 |
| 13 | 17.444 | 5.07991 | 52.3168 | 547.243 | 5.0 |
| 14 | 18.654 | 4.75303 | 220.011 | 734.270 | 20.9 |
| 15 | 19.075 | 4.65889 | 386.326 | 907.044 | 36.8 |
| 16 | 19.315 | 4.59168 | 355.661 | 879.275 | 33.8 |
| 17 | 20.086 | 4.41715 | 280.044 | 809.118 | 26.6 |
| 18 | 20.669 | 4.29396 | 487.007 | 1016.32 | 46.3 |
| 19 | 21.229 | 4.18186 | 701.484 | 1227.88 | 66.7 |
| 20 | 21.426 | 4.14394 | 883.346 | 1407.98 | 84.0 |
| 21 | 22.195 | 4.00202 | 686.478 | 1200.58 | 65.3 |
| 22 | 23.118 | 3.84426 | 511.216 | 1004.99 | 48.6 |
| 23 | 23.362 | 3.80467 | 537.891 | 1024.90 | 51.2 |
| 24 | 24.433 | 3.64028 | 150.351 | 600.683 | 14.3 |
| 25 | 25.086 | 3.54698 | 122.546 | 544.974 | 11.7 |
| 26 | 26.850 | 3.31785 | 217.435 | 577.303 | 20.7 |
| 27 | 27.391 | 3.25345 | 79.9943 | 428.410 | 7.6 |
| 28 | 27.932 | 3.19173 | 58.7867 | 392.904 | 5.6 |
| 29 | 28.921 | 3.08474 | 59.6694 | 380.069 | 5.7 |
| 30 | 29.628 | 3.01269 | 56.6703 | 369.640 | 5.4 |
| 31 | 31.016 | 2.88101 | 54.4981 | 340.968 | 5.2 |
| 32 | 31.343 | 2.85164 | 60.8633 | 338.310 | 5.8 |
| 33 | 33.937 | 2.63937 | 58.5601 | 292.580 | 5.6 |

The X-ray powder diffraction pattern of crystalline form B of compound ① is shown in FIG. 2.

### Example 4: Preparation of crystalline form C of compound ①

Compound ① (5.1 g) was collected and dissolved in IPAc (isopropyl acetate) (5 mL) at room temperature. The IPAc solution of compound ① was dropwise added slowly into n-hexane (120 mL) while maintaining the temperature at room temperature. After the addition was completes, the mixture was heated to 60 °C and stirred for dissolution. Then the heating was switched off and cooled naturally to room temperature to precipitate white flocculent solid. The solid was filtered and the filter cake was dried under vacuum at 55 °C to obtain crystalline form C.

### X-ray powder diffraction analysis:

Crystalline form C of compound ① was characterized by X-ray diffractometer (Bruker D8 advance) using graphite-monochromated Cu Kα radiation (λ = 1.54) at room temperature. Method parameters are as follows: current voltage: -40 mA, 40 Kv; step length: 0.02°; scanning rate: 0.2 sec/step; scanning range: 3°- 40°2θ. The powder diffraction pattern was obtained, and data were analyzed. The X-ray powder diffraction data of crystalline form C are shown in Table 4.

**Table 4: X-ray powder diffraction data of crystalline form C of compound (1)**

| Peak No. | 2θ Angle (°) | Interplanar Spacing | Peak Height | Half Peak Width | Relative Peak Height (%) |
|---|---|---|---|---|---|
| 1 | 6.790 | 13.00738 | 240.982 | 612.075 | 33.6 |
| 2 | 9.917 | 8.91160 | 436.447 | 813.771 | 60.9 |
| 3 | 10.551 | 8.37750 | 89.9581 | 498.499 | 12.5 |
| 4 | 11.533 | 7.66644 | 445.866 | 893.380 | 62.2 |
| 5 | 12.600 | 7.01947 | 190.324 | 667.285 | 26.5 |
| 6 | 13.556 | 6.52664 | 363.746 | 855.653 | 50.7 |
| 7 | 13.793 | 6.41524 | 207.245 | 701.185 | 28.9 |
| 8 | 14.275 | 6.19954 | 170.742 | 666.783 | 23.8 |
| 9 | 15.008 | 5.89843 | 156.695 | 674.482 | 21.8 |
| 10 | 15.912 | 5.56518 | 106.030 | 660.383 | 14.8 |
| 11 | 16.452 | 5.38381 | 257.378 | 828.947 | 35.9 |
| 12 | 16.941 | 5.22940 | 87.1279 | 671.334 | 12.1 |
| 13 | 17.877 | 4.95765 | 450.838 | 1063.77 | 62.9 |
| 14 | 19.066 | 4.65110 | 535.650 | 1186.86 | 74.7 |
| 15 | 19.774 | 4.48627 | 354.795 | 1020.86 | 49.5 |
| 16 | 20.300 | 4.37101 | 661.429 | 1334.72 | 92.2 |
| 17 | 20.978 | 4.23124 | 717.225 | 1394.99 | 100.0 |
| 18 | 21.616 | 4.10788 | 651.879 | 1328.89 | 90.9 |
| 19 | 22.585 | 3.93369 | 362.113 | 1028.78 | 50.5 |
| 20 | 23.407 | 3.79745 | 382.646 | 1031.86 | 53.4 |
| 21 | 25.877 | 3.44032 | 84.2856 | 640.036 | 11.8 |
| 22 | 28.238 | 3.15774 | 49.6080 | 486.235 | 6.9 |
| 23 | 30.621 | 2.91726 | 108.816 | 472.516 | 15.2 |
| 24 | 30.838 | 2.89722 | 65.3740 | 421.254 | 9.1 |

The X-ray powder diffraction pattern of crystalline form C of compound ① is shown in FIG. 3.

### Example 5: Preparation of crystalline form D of compound ①

The crude product was prepared by reversed-phase purification with acetonitrile and water, then concentrated, and extracted with ethyl acetate, then concentrated again, and then dried under vacuum at 55 °C to obtain off-white solid compound ① (34 g), which was found to be crystalline form D by XRD.

### X-ray powder diffraction analysis:

The crystalline form D of compound ① was characterized by X-ray diffractometer (Bruker D8 advance) using graphite-monochromated Cu Kα radiation (λ = 1.54) at room temperature. Method parameters are as follows: current voltage: -40 mA, 40 Kv; step length: 0.02°; scanning rate: 0.2 sec/step; scanning range: 3°- 40°2θ. The powder diffraction pattern was obtained, and data were analyzed. The X-ray powder diffraction data of crystalline form D are shown in Table 5.

**Table 5: X-ray powder diffraction data of crystalline form D of compound (1)**

| Peak No. | 2θ Angle (°) | Interplanar Spacing | Peak Height | Half Peak Width | Relative Peak Height (%) |
|---|---|---|---|---|---|
| 1 | 5.000 | 17.65804 | 519.142 | 1003.63 | 8.0 |
| 2 | 6.646 | 13.28987 | 4520.01 | 4959.97 | 69.7 |
| 3 | 8.920 | 9.90524 | 93.4109 | 523.481 | 1.4 |
| 4 | 9.869 | 8.95507 | 1167.24 | 1625.83 | 18.0 |
| 5 | 10.255 | 8.61898 | 91.6051 | 568.602 | 1.4 |
| 6 | 11.340 | 7.79661 | 493.671 | 996.433 | 7.5 |
| 7 | 11.859 | 7.45634 | 197.196 | 718.727 | 3.0 |
| 8 | 12.638 | 6.99866 | 838.576 | 1397.22 | 12.9 |
| 9 | 13.175 | 6.71478 | 6488.71 | 7073.40 | 100.0 |
| 10 | 13.489 | 6.55875 | 1700.32 | 2297.59 | 26.2 |
| 11 | 14.165 | 6.24744 | 232.638 | 850.237 | 3.6 |
| 12 | 14.870 | 5.95264 | 173.095 | 802.156 | 2.7 |
| 13 | 15.555 | 5.69212 | 124.800 | 786.784 | 1.9 |
| 14 | 16.042 | 5.52047 | 719.118 | 1408.06 | 11.1 |
| 15 | 16.450 | 5.38455 | 1549.64 | 2257.51 | 23.9 |
| 16 | 17.328 | 5.11347 | 217.791 | 976.556 | 3.4 |
| 17 | 17.583 | 5.03982 | 373.357 | 1146.94 | 5.8 |
| 18 | 18.185 | 4.87447 | 608.362 | 1415.79 | 9.4 |
| 19 | 18.808 | 4.71429 | 172.619 | 1009.21 | 2.7 |
| 20 | 19.225 | 4.61299 | 451.149 | 1302.89 | 7.0 |
| 21 | 19.562 | 4.53428 | 672.101 | 1533.55 | 10.4 |
| 22 | 19.807 | 4.47870 | 807.190 | 1674.26 | 12.4 |
| 23 | 20.371 | 4.35599 | 435.293 | 1310.74 | 6.7 |
| 24 | 20.950 | 4.23689 | 1394.77 | 2272.20 | 21.5 |
| 25 | 21.585 | 4.11377 | 2212.26 | 3084.13 | 34.1 |
| 26 | 22.369 | 3.97131 | 2183.42 | 3037.26 | 33.6 |
| 27 | 23.036 | 3.85775 | 1727.18 | 2555.99 | 26.6 |
| 28 | 23.281 | 3.81777 | 1704.69 | 2522.09 | 26.3 |
| 29 | 24.141 | 3.68368 | 495.404 | 1263.16 | 7.6 |
| 30 | 24.850 | 3.58013 | 228.252 | 940.918 | 3.5 |
| 31 | 25.392 | 3.50495 | 353.338 | 1022.41 | 5.4 |
| 32 | 25.764 | 3.45514 | 65.8463 | 709.413 | 1.0 |
| 33 | 26.519 | 3.35844 | 272.556 | 888.475 | 4.2 |
| 34 | 27.005 | 3.29910 | 326.830 | 918.919 | 5.0 |
| 35 | 27.668 | 3.22150 | 116.565 | 668.482 | 1.8 |
| 36 | 27.969 | 3.18771 | 127.278 | 658.180 | 2.0 |
| 37 | 29.121 | 3.06404 | 76.2929 | 542.059 | 1.2 |
| 38 | 29.613 | 3.01420 | 785.567 | 1255.31 | 12.1 |
| 39 | 30.115 | 2.96513 | 209.518 | 678.315 | 3.2 |
| 40 | 30.956 | 2.88641 | 153.699 | 609.577 | 2.4 |
| 41 | 31.490 | 2.83869 | 46.8668 | 487.194 | 0.7 |
| 42 | 32.570 | 2.74701 | 151.866 | 582.158 | 2.3 |
| 43 | 32.798 | 2.72839 | 145.395 | 574.945 | 2.2 |
| 44 | 34.247 | 2.61623 | 89.3799 | 497.906 | 1.4 |
| 45 | 35.528 | 2.52478 | 60.3312 | 422.563 | 0.9 |
| 46 | 36.148 | 2.48290 | 69.1802 | 407.510 | 1.1 |

The X-ray powder diffraction pattern of crystalline form D of compound ① is shown in FIG. 4.

### Example 6: Preparation of crystalline form E of compound ①

The compound ① (2.0 g) was added into a reaction flask, ethyl acetate (4 mL) was added at room temperature and stirred to dissolve at 60 °C, n-hexane (6 mL) was added while maintaining internal temperature at 60 °C and continuously stirring for 2 hours. A large amount of white floccules appeared, the floccules were filtered, and the obtained filter cake was vacuum-dried at 55 °C to obtain crystalline form E.

### X-ray powder diffraction analysis:

The crystalline form E of compound ① was characterized by X-ray diffractometer (Bruker D8 advance) using graphite-monochromated Cu Kα radiation (λ = 1.54) at room temperature. Method parameters are as follows: current voltage: -40 mA, 40 Kv; step length: 0.02°; scanning rate: 0.2 sec/step; scanning range: 3°- 40°2θ. The powder diffraction pattern was obtained, and data were analyzed. The X-ray powder diffraction data of crystalline form E are shown in Table 6.

**Table 6: X-ray powder diffraction data of crystalline form E**

| Peak No. | 2θ Angle (°) | Interplanar Spacing | Peak Height | Half Peak Width | Relative Peak Height (%) |
|---|---|---|---|---|---|
| 1 | 4.955 | 17.81808 | 415.303 | 855.689 | 9.0 |
| 2 | 6.106 | 14.46322 | 399.812 | 809.667 | 8.7 |
| 3 | 8.129 | 10.86709 | 833.962 | 1255.08 | 18.1 |
| 4 | 9.489 | 9.31289 | 228.693 | 673.241 | 5.0 |
| 5 | 11.118 | 7.95215 | 726.088 | 1237.08 | 15.8 |
| 6 | 11.360 | 7.78299 | 683.785 | 1202.96 | 14.8 |
| 7 | 11.749 | 7.52585 | 1224.73 | 1754.96 | 26.6 |
| 8 | 12.065 | 7.32992 | 196.946 | 734.201 | 4.3 |
| 9 | 12.700 | 6.96472 | 209.517 | 755.726 | 4.6 |
| 10 | 13.482 | 6.56243 | 758.947 | 1306.64 | 16.5 |
| 11 | 14.730 | 6.00908 | 1663.98 | 2226.96 | 36.1 |
| 12 | 15.256 | 5.80300 | 434.647 | 1016.00 | 9.4 |
| 13 | 16.369 | 5.41099 | 385.387 | 989.908 | 8.4 |
| 14 | 17.354 | 5.10589 | 59.0790 | 696.464 | 1.3 |
| 15 | 18.227 | 4.86327 | 2414.96 | 3108.97 | 52.4 |
| 16 | 19.073 | 4.64955 | 127.661 | 864.010 | 2.8 |
| 17 | 19.362 | 4.58077 | 277.403 | 1025.40 | 6.0 |
| 18 | 19.954 | 4.44616 | 4604.65 | 5372.02 | 100.0 |
| 19 | 20.712 | 4.28512 | 1378.69 | 2162.03 | 29.9 |
| 20 | 21.092 | 4.20870 | 735.185 | 1522.82 | 16.0 |
| 21 | 21.400 | 4.14887 | 388.856 | 1178.13 | 8.4 |
| 22 | 22.449 | 3.95736 | 2349.36 | 3131.99 | 51.0 |
| 23 | 22.813 | 3.89491 | 535.644 | 1311.53 | 11.6 |
| 24 | 23.659 | 3.75754 | 163.549 | 924.723 | 3.6 |
| 25 | 24.399 | 3.64526 | 762.480 | 1512.85 | 16.6 |
| 26 | 24.560 | 3.62170 | 839.335 | 1586.09 | 18.2 |
| 27 | 25.253 | 3.52388 | 285.987 | 1012.15 | 6.2 |
| 28 | 25.923 | 3.43427 | 698.967 | 1397.34 | 15.2 |
| 29 | 26.429 | 3.36963 | 586.940 | 1259.20 | 12.7 |
| 30 | 27.060 | 3.29250 | 464.489 | 1098.02 | 10.1 |
| 31 | 27.446 | 3.24712 | 553.589 | 1160.08 | 12.0 |
| 32 | 28.020 | 3.18183 | 144.118 | 705.553 | 3.1 |
| 33 | 29.708 | 3.00480 | 206.550 | 709.041 | 4.5 |
| 34 | 30.131 | 2.96360 | 155.774 | 648.686 | 3.4 |
| 35 | 30.694 | 2.91049 | 81.5822 | 562.943 | 1.8 |
| 36 | 31.368 | 2.84944 | 139.165 | 607.243 | 3.0 |
| 37 | 32.127 | 2.78389 | 208.190 | 651.976 | 4.5 |
| 38 | 32.906 | 2.71973 | 162.042 | 570.552 | 3.5 |
| 39 | 33.368 | 2.68308 | 187.943 | 570. 564 | 4.1 |
| 40 | 35.142 | 2.55164 | 43.6476 | 394.460 | 0.9 |
| 41 | 35.935 | 2.49708 | 60.9710 | 422.245 | 1.3 |
| 42 | 37.809 | 2.37754 | 80.3217 | 443.244 | 1.7 |
| 43 | 38.353 | 2.34507 | 42.9184 | 395.392 | 0.9 |

The X-ray powder diffraction pattern of crystalline form E of compound ① is shown in FIG. 5.

### Example 7: Preparation of crystalline form F of compound ①

Compound ① (500 mg) was weighed and placed into 50 mL reaction flask, 2 mL of *n-*propanol was added and heated to reflux and dissolve, 2 mL of n-heptane was dropped, then waited for solution to become clear again, then cooled to room temperature for crystallization for 30 minutes, then filtered, and the filter cake was vacuum-dried at 55 °C for 9 hours to obtain crystalline form F (420 mg).

### X-ray powder diffraction analysis:

Crystalline form F of compound ① was characterized by X-ray diffractometer (Bruker D8 advance) using graphite-monochromated Cu Kα radiation (λ = 1.54) at room temperature. Method parameters are as follows: current voltage: -40 mA, 40 Kv; step length: 0.02°; scanning rate: 0.2 sec/step; scanning range: 3°- 40°2θ. The powder diffraction pattern was obtained, and data were analyzed. The X-ray powder diffraction data of crystalline form F are shown in Table 7.

**Table 7: X-ray powder diffraction data of crystalline form F**

| Peak No. | 2θ Angle (°) | Interplanar Spacing | Peak Height | Half Peak Width | Relative Peak Height (%) |
|---|---|---|---|---|---|
| 1 | 6.717 | 13.14950 | 3128.30 | 3478.01 | 88.3 |
| 2 | 9.812 | 9.00713 | 614.584 | 965.350 | 17.3 |
| 3 | 10.067 | 8.77952 | 147.069 | 503.389 | 4.2 |
| 4 | 10.502 | 8.41646 | 83.6918 | 447.243 | 2.4 |
| 5 | 10.702 | 8.25988 | 52.5999 | 418.514 | 1.5 |
| 6 | 11.426 | 7.73829 | 793.138 | 1162.62 | 22.4 |
| 7 | 12.501 | 7.07521 | 574.498 | 964.319 | 16.2 |
| 8 | 13.484 | 6.56131 | 3543.02 | 3968.31 | 100.0 |
| 9 | 14.199 | 6.23273 | 245.355 | 687.348 | 6.9 |
| 10 | 14.915 | 5.93491 | 369.779 | 820.827 | 10.4 |
| 11 | 15.144 | 5.84569 | 183.155 | 635.476 | 5.2 |
| 12 | 15.749 | 5.62234 | 91.3901 | 552.380 | 2.6 |
| 13 | 16.374 | 5.40911 | 459.916 | 934.800 | 13.0 |
| 14 | 16.884 | 5.24706 | 640.597 | 1122.48 | 18.1 |
| 15 | 17.828 | 4.97113 | 656.939 | 1141.51 | 18.5 |
| 16 | 18.975 | 4.67311 | 978.922 | 1479.24 | 27.6 |
| 17 | 19.703 | 4.50211 | 343.527 | 856.725 | 9.7 |
| 18 | 20.241 | 4.38366 | 1291.08 | 1808.70 | 36.4 |
| 19 | 20.902 | 4.24646 | 1377.77 | 1894.89 | 38.9 |
| 20 | 21.593 | 4.11226 | 960.803 | 1470.41 | 27.1 |
| 21 | 22.506 | 3.94745 | 629.907 | 1118.61 | 17.8 |
| 22 | 22.918 | 3.87736 | 367.060 | 842.238 | 10.4 |
| 23 | 23.412 | 3.79658 | 428.937 | 884.530 | 12.1 |
| 24 | 23.735 | 3.74575 | 305.117 | 745.973 | 8.6 |
| 25 | 24.019 | 3.70199 | 273.549 | 700.096 | 7.7 |
| 26 | 24.337 | 3.65445 | 96.6887 | 505.857 | 2.7 |
| 27 | 25.595 | 3.47753 | 164.786 | 514.024 | 4.7 |
| 28 | 25.779 | 3.45321 | 266.436 | 606.681 | 7.5 |
| 29 | 26.218 | 3.39635 | 86.3708 | 403.021 | 2.4 |
| 30 | 27.245 | 3.27056 | 67.5274 | 361.092 | 1.9 |
| 31 | 28.185 | 3.16363 | 32.2744 | 316.254 | 0.9 |
| 32 | 28.388 | 3.14140 | 89.8839 | 375.507 | 2.5 |
| 33 | 28.786 | 3.09893 | 161.028 | 448.067 | 4.5 |
| 34 | 30.510 | 2.92757 | 109.478 | 375.259 | 3.1 |
| 35 | 30.713 | 2.90868 | 116.520 | 376.868 | 3.3 |
| 36 | 32.016 | 2.79326 | 58.0219 | 273.413 | 1.6 |
| 37 | 33.537 | 2.66997 | 66.1289 | 280.026 | 1.9 |
| 38 | 33.778 | 2.65144 | 78.3998 | 294.551 | 2.2 |
| 39 | 35.164 | 2.55008 | 68.2875 | 280.462 | 1.9 |

The X-ray powder diffraction pattern of crystalline form F of compound ① is shown in FIG. 6.

### TGA and DSC analysis:

Applying test conditions of crystalline form A of compound ①, crystalline form F of compound ① was subjected to the thermogravimetric analysis (TGA) and differential scanning calorimetry (DSC) respectively. The test results are shown in FIG. 12 and 13 respectively. The TGA thermogram shows a weight increase of 0.697% when heated to 105 °C. DSC thermogram shows an endothermic peak value of 126.78 °C.

### Example 8: Preparation of crystalline form G of compound ①

Compound ① (500 mg) was placed into a 50 mL reaction flask and 2 mL of ethanol was added. The temperature was raised to reflux, and after the solution was dissolved, 2 mL of *n-*heptane was added drop by drop. After solution was clear again, the temperature was lowered to room temperature and stirred for 60 minutes for crystallization. The obtained mixture was filtered and the filter cake was vacuum-dried at 55 °C for 9 hours to obtain 350 mg of white solid (crystalline form G of compound ①).

### X-ray powder diffraction analysis:

Crystalline form G of compound ① was characterized by X-ray diffractometer (Bruker D8 advance) using graphite-monochromated Cu Kα radiation (λ = 1.54) at room temperature. Method parameters are as follows: current voltage: -40 mA, 40 Kv; step length: 0.02°; scanning rate: 0.2 sec/step; scanning range: 3°- 40°2θ. The powder diffraction pattern was obtained, and data were analyzed. The X-ray powder diffraction data of crystalline form G are shown in Table 8.

**Table 8: X-ray powder diffraction data of crystalline form G**

| Peak No. | 2θ Angle (°) | Interplanar Spacing | Peak Height | Half Peak Width | Relative Peak Height (%) |
|---|---|---|---|---|---|
| 1 | 6.743 | 13.09752 | 6955.11 | 7322.73 | 82.2 |
| 2 | 7.172 | 12.31633 | 82.3872 | 446.157 | 1.0 |
| 3 | 9.661 | 9.14789 | 136.255 | 498.600 | 1.6 |
| 4 | 9.830 | 8.99057 | 255.518 | 620.828 | 3.0 |
| 5 | 10.118 | 8.73530 | 323.729 | 692.594 | 3.8 |
| 6 | 10.720 | 8.24635 | 35.2406 | 405.507 | 0.4 |
| 7 | 11.448 | 7.72310 | 515.082 | 882.288 | 6.1 |
| 8 | 12.516 | 7.06650 | 373.446 | 766.037 | 4.4 |
| 9 | 12.710 | 6.95923 | 118.354 | 520.537 | 1.4 |
| 10 | 13.503 | 6.55222 | 8465.96 | 8898.57 | 100.0 |
| 11 | 13.896 | 6.36788 | 34.613 | 767.047 | 3.8 |
| 12 | 14.201 | 6.23178 | 142.963 | 590.628 | 1.7 |
| 13 | 14.982 | 5.90848 | 311.094 | 769.301 | 3.7 |
| 14 | 15.178 | 5.83256 | 250.982 | 710.953 | 3.0 |
| 15 | 15.783 | 5.61032 | 79.2374 | 548.014 | 0.9 |
| 16 | 16.396 | 5.40189 | 533.422 | 1014.76 | 6.3 |
| 17 | 16.901 | 5.24166 | 1609.14 | 2094.47 | 19.0 |
| 18 | 17.415 | 5.08821 | 141.271 | 624.747 | 1.7 |
| 19 | 17.865 | 4.96091 | 654.078 | 1131.04 | 7.7 |
| 20 | 18.996 | 4.66802 | 932.146 | 1429.40 | 11.0 |
| 21 | 19.343 | 4.58523 | 158.053 | 667.076 | 1.9 |
| 22 | 19.700 | 4.50276 | 269.561 | 787.911 | 3.2 |
| 23 | 20.260 | 4.37974 | 1422.16 | 1949.31 | 16.8 |
| 24 | 20.920 | 4.24287 | 1392.94 | 1921.41 | 16.5 |
| 25 | 21.604 | 4.11003 | 908.939 | 1428.39 | 10.7 |
| 26 | 22.513 | 3.94610 | 563.624 | 1054.78 | 6.7 |
| 27 | 22.929 | 3.87555 | 329.614 | 801.638 | 3.9 |
| 28 | 23.469 | 3.78752 | 365.940 | 807.245 | 4.3 |
| 29 | 23.744 | 3.74428 | 552.245 | 975.390 | 6.5 |
| 30 | 24.033 | 3.70000 | 398.621 | 800.892 | 4.7 |
| 31 | 24.363 | 3.65049 | 141.439 | 517.452 | 1.7 |
| 32 | 25.268 | 3.52185 | 34.3551 | 362.195 | 0.4 |
| 33 | 25.606 | 3.47611 | 232.235 | 550.635 | 2.7 |
| 34 | 25.810 | 3.44913 | 353.200 | 664.663 | 4.2 |
| 35 | 26.260 | 3.39102 | 74.0289 | 366.854 | 0.9 |
| 36 | 26.786 | 3.32556 | 48.9860 | 330.568 | 0.6 |
| 37 | 27.236 | 3.27167 | 115.839 | 390.882 | 1.4 |
| 38 | 28.138 | 3.16879 | 50.7672 | 314.451 | 0.6 |
| 39 | 28.379 | 3.14240 | 98.6973 | 365.283 | 1.2 |
| 40 | 28.815 | 3.09582 | 237.517 | 506.029 | 2.8 |
| 41 | 29.306 | 3.04505 | 89.0091 | 354.544 | 1.1 |
| 42 | 30.484 | 2.93010 | 144.463 | 395.649 | 1.7 |
| 43 | 30.797 | 2.90101 | 157.270 | 403.538 | 1.9 |
| 44 | 31.976 | 2.79668 | 55.1173 | 267.886 | 0.7 |
| 45 | 33.531 | 2.67044 | 97.0757 | 309.549 | 1.1 |
| 46 | 33.798 | 2.64991 | 133.231 | 347.962 | 1.6 |
| 47 | 34.175 | 2.62158 | 45.8969 | 261.080 | 0.5 |
| 48 | 34.538 | 2.59482 | 41.8055 | 254.377 | 0.5 |
| 49 | 35.167 | 2.54988 | 89.8113 | 290.856 | 1.1 |
| 50 | 38.912 | 2.31261 | 77.4143 | 247.923 | 0.9 |

The X-ray powder diffraction pattern of crystalline form G of compound ① is shown in FIG. 7.

### Example 9: Preparation of crystalline form H of compound ①

The crude product of compound ① (2.2 kg) was placed in a reactor, acetonitrile (4.1 kg) was added at the room temperature and stirred to dissolve it, then water (7.8 kg) was added and stirred for crystallization for 20 hours, 5 L of acetonitrile/H₂O mixed solvent (mixed solvent ratio: acetonitrile 4.1 kg + H₂O 7.8 kg) was added, stirring was maintained for 1 hour and then the mixture was centrifuged, the filter cake was vacuum-dried at 55 °C to obtain crystalline form H of compound ① (1.54 kg).

### X-ray powder diffraction analysis:

The crystalline form H of compound ① was characterized by X-ray diffractometer (Bruker D8 advance) using graphite-monochromated Cu Kα radiation (λ = 1.54) at room temperature. Method parameters are as follows: current voltage: -40 mA, 40 Kv; step length: 0.02°; scanning rate: 0.2 sec/step; scanning range: 3°- 40°2θ. The powder diffraction pattern was obtained, and data were analyzed. The X-ray powder diffraction data of crystalline form H are shown in Table 9.

**Table 9: X-ray powder diffraction data of crystalline form H**

| Peak No. | 2θ Angle (°) | Interplanar Spacing | Peak Height | Half Peak Width | Relative Peak Height (%) |
|---|---|---|---|---|---|
| 1 | 6.514 | 13.55902 | 1011.66 | 1482.87 | 43.8 |
| 2 | 10.074 | 8.77369 | 983.689 | 1536.17 | 42.6 |
| 3 | 10.684 | 8.27358 | 1269.58 | 1849.10 | 55.0 |
| 4 | 11.433 | 7.73331 | 104.602 | 710.730 | 4.5 |
| 5 | 12.366 | 7.15226 | 1535.62 | 2164.81 | 66.5 |
| 6 | 13.115 | 6.74502 | 1276.52 | 1916.16 | 55.3 |
| 7 | 14.359 | 6.16368 | 1267.70 | 1926.79 | 54.9 |
| 8 | 15.617 | 5.66979 | 2309.33 | 2989.38 | 100.0 |
| 9 | 16.909 | 5.23937 | 1560.43 | 2240.84 | 67.6 |
| 10 | 18.305 | 4.84280 | 425.604 | 1138.42 | 18.4 |
| 11 | 19.147 | 4.63155 | 197.987 | 962.156 | 8.6 |
| 12 | 19.839 | 4.47162 | 1100.22 | 1899.72 | 47.6 |
| 13 | 20.092 | 4.41594 | 1662.30 | 2473.18 | 72.0 |
| 14 | 20.687 | 4.29017 | 1403.38 | 2237.82 | 60.8 |
| 15 | 22.919 | 3.87716 | 1348.88 | 2231.12 | 58.4 |
| 16 | 24.006 | 3.70402 | 388.325 | 1270.67 | 16.8 |
| 17 | 25.749 | 3.45714 | 414.621 | 1265.44 | 18.0 |
| 18 | 27.318 | 3.26200 | 340.101 | 1129.15 | 14.7 |
| 19 | 27.741 | 3.21324 | 214.440 | 981.435 | 9.3 |
| 20 | 29.177 | 3.05821 | 194.893 | 872.631 | 8.4 |
| 21 | 29.802 | 2.99552 | 365.341 | 1002.94 | 15.8 |
| 22 | 32.803 | 2.72799 | 72.8044 | 583.628 | 3.2 |

The X-ray powder diffraction pattern of crystalline form H of compound ① is shown in FIG. 8.

### TGA and DSC analysis:

Applying the test conditions of the crystalline form A of compound ①, the crystalline form H of compound ① was subj ected to thermogravimetric analysis (TGA) as well as differential scanning calorimetry (DSC) respectively. Test results are shown in FIG. 14 and 15 respectively. The TGA thermogram shows a weight loss of 0.6674% upon heating to 101.42 °C; the DSC thermogram shows an endothermic peak value of 130.05 °C.

### Example 10: Preparation of crystalline form I of compound ①

The crude product of compound ① (60 g) was placed into a reaction flask, acetonitrile (120 mL) was added at room temperature and stirred to dissolve, water (180 mL) was then added and stirred for crystallization for 3 hours, then acetonitrile (120 mL)/H₂O (180 mL) mixed solvent was added, crystallization was continued for 2 hours, and the mixture as filtered and then the filter cake was vacuum-dried at 55 °C to obtain crystalline form I (40.5 g).

### X-ray powder diffraction analysis:

Crystalline form I of compound ① was characterized by X-ray diffractometer (Bruker D8 advance) using graphite-monochromated Cu Kα radiation (λ = 1.54) at room temperature. Method parameters are as follows: current voltage: -40 mA, 40 Kv; step length: 0.02°; scanning rate: 0.2 sec/step; scanning range: 3°- 40°2θ. The powder diffraction pattern was obtained, and data were analyzed. X-ray powder diffraction data of crystalline form I are shown in Table 10.

**Table 10: X-ray powder diffraction data of crystalline form I**

| Peak No. | 2θ Angle (°) | Interplanar Spacing | Peak Height | Half Peak Width | Relative Peak Height (%) |
|---|---|---|---|---|---|
| 1 | 4.968 | 17.77162 | 5143.76 | 5677.95 | 100.0 |
| 2 | 8.863 | 9.96933 | 1558.15 | 1990.41 | 30.3 |
| 3 | 9.895 | 8.93159 | 881.271 | 1345.81 | 17.1 |
| 4 | 10.224 | 8.64522 | 698.937 | 1172.59 | 13.6 |
| 5 | 10.603 | 8.33713 | 369.419 | 851.065 | 7.2 |
| 6 | 11.841 | 7.46761 | 743.075 | 1268.43 | 14.4 |
| 7 | 12.739 | 6.94315 | 1465.12 | 2047.07 | 28.5 |
| 8 | 13.167 | 6.71847 | 951.656 | 1555.22 | 18.5 |
| 9 | 13.896 | 6.36772 | 1375.98 | 2008.41 | 26.8 |
| 10 | 14.234 | 6.21740 | 462.608 | 1105.01 | 9.0 |
| 11 | 14.676 | 6.03106 | 258.453 | 910.675 | 5.0 |
| 12 | 14.848 | 5.96148 | 1281.30 | 1936.34 | 24.9 |
| 13 | 15.229 | 5.81334 | 271.102 | 930.408 | 5.3 |
| 14 | 16.177 | 5.47466 | 409.307 | 1067.35 | 8.0 |
| 15 | 16.530 | 5.35868 | 72.2338 | 725.471 | 1.4 |
| 16 | 17.349 | 5.10734 | 402.953 | 1071.72 | 7.8 |
| 17 | 17.743 | 4.99490 | 297.555 | 991.060 | 5.8 |
| 18 | 18.203 | 4.86965 | 600.703 | 1320.78 | 11.7 |
| 19 | 18.840 | 4.70643 | 1578.88 | 2330.26 | 30.7 |
| 20 | 19.179 | 4.62391 | 2500.06 | 3265.01 | 48.6 |
| 21 | 19.770 | 4.48708 | 3800.51 | 4583.86 | 73.9 |
| 22 | 20.232 | 4.38571 | 229.055 | 1022.22 | 4.5 |
| 23 | 20.491 | 4.33074 | 526.429 | 1323.35 | 10.2 |
| 24 | 20.853 | 4.25646 | 450.554 | 1250.58 | 8.8 |
| 25 | 21.155 | 4.19632 | 1706.83 | 2507.56 | 33.2 |
| 26 | 21.752 | 4.08240 | 3723.80 | 4520.86 | 72.4 |
| 27 | 22.082 | 4.02218 | 357.542 | 1149.69 | 7.0 |
| 28 | 22.465 | 3.95450 | 664.408 | 1448.28 | 12.9 |
| 29 | 22.985 | 3.86627 | 74.0109 | 843.106 | 1.4 |
| 30 | 23.629 | 3.76230 | 811.018 | 1559.92 | 15.8 |
| 31 | 24.053 | 3.69693 | 1461.42 | 2192.77 | 28.4 |
| 32 | 24.647 | 3.60918 | 258.445 | 959.496 | 5.0 |
| 33 | 24.920 | 3.57023 | 618.695 | 1303.58 | 12.0 |
| 34 | 25.703 | 3.46317 | 874.631 | 1505.34 | 17.0 |
| 35 | 26.437 | 3.36873 | 108.589 | 690.804 | 2.1 |
| 36 | 26.870 | 3.31539 | 533.222 | 1090.61 | 10.4 |
| 37 | 27.188 | 3.27726 | 198.957 | 735.228 | 4.8 |
| 38 | 27.741 | 3.21318 | 123.212 | 626.480 | 2.4 |
| 39 | 27.958 | 3.18878 | 245.526 | 735.228 | 4.8 |
| 40 | 28.280 | 3.15316 | 51.8301 | 519.700 | 1.0 |
| 41 | 28.615 | 3.11708 | 53.9955 | 497.147 | 1.0 |
| 42 | 28.905 | 3.08647 | 109.477 | 539.861 | 2.1 |
| 43 | 29.128 | 3.06327 | 389.472 | 817.329 | 7.6 |
| 44 | 29.525 | 3.02301 | 197.330 | 618.389 | 3.8 |
| 45 | 29.904 | 2.98553 | 431.934 | 843.724 | 8.4 |
| 46 | 20.546 | 2.92423 | 183.047 | 572.947 | 3.6 |

The X-ray powder diffraction pattern of crystalline form I of compound ① is shown in FIG. 9.

### Biological Embodiments

### 1. In vivo pharmacokinetic studies

A proper amount of compound was weighed and 1 mg/mL suspension was prepared using the weighed compound and 5% DMSO + 30% HP-β-CD. After ICR male mice fasted overnight, they were administered by gavage and their blood was collected from the jugular venous plexus at different time points of 0, 5 min, 0.25 h, 0.5 h, 1 h, 2 h, 4 h, 8 h, and 24 h. After anticoagulation with EDTA-K2, plasma was separated by centrifugation, and the concentrations of prototype drug in plasma was determined by LC/MS/MS.

Results show that crystalline form A, crystalline form B, crystalline form C, crystalline form D, crystalline form E, crystalline form F, crystalline form G, crystalline form H and crystalline form I of compound ① all have good pharmacokinetic properties.

### 2. Stability Study

### 2.1 Influencing factors test

### 2.1.1 Lighting test

A proper amount of compound was weighed and placed in a suitable white transparent open container (such as a weighing bottle or petri dish), the container was placed in a lighting stability test chamber for drugs at an illumination of 4,500 lx ± 500 lx. The samples were taken for testing 5, 10, and 30 days after the placement.

### 2.1.2 High-temperature test

A proper amount of compound was weighed and placed in a suitable open container (such as a weighing bottle or culture dish), the container was placed in an electric heated air-drying oven at 60°C. The samples were taken for testing 5, 10, and 30 days after the placement.

### 2.1.3 High-humidity test

A proper amount of compound was weighed and placed in suitable open container (such as a weighing bottle or petri dish), the container was placed in a KNO₃ saturated solution desiccator at a temperature of 25 °C ± 2 °C and a relative humidity of 92.5% and 72.5% respectively. The samples were taken for testing 5, 10, and 30 days after the placement.

### 2.2 Acceleration test

After packaging an appropriate amount of the compound with inner packaging (pharmaceutical low-density polyethylene film, bag) and outer packaging (polyester/aluminum/polyethylene pharmaceutical composite film, bag), the compound was tested at a temperature of 40 °C ± 2 °C and a relative humidity of 75% ± 5%.

### 2.3 Intermediate conditions

After packaging an appropriate amount of compound with inner packaging (pharmaceutical low-density polyethylene film, bag) as well as outer packaging (polyester/aluminum/polyethylene pharmaceutical composite film, bag), the compound was tested at a temperature of 30 °C ± 2 °C and a relative humidity of 65% ± 5%.

### 2.4 Long-term test

After packaging an appropriate amount of compound with inner packaging (pharmaceutical low-density polyethylene film, bag) as well as outer packaging (polyester/aluminum/polyethylene pharmaceutical composite film, bag), the compound was tested at a temperature of 25 °C ± 2 °C and a relative humidity of 60% ± 5%.

Results show that crystalline form A, crystalline form B, crystalline form C, crystalline form D, crystalline form E, crystalline form F, crystalline form G, crystalline form H and crystalline form I of compound ① all have good physical and chemical stability.

The specification of the present invention describes the specific embodiments in detail. Those skilled in the art should recognize that the above embodiments are exemplary and cannot be understood as limiting the present invention. For those skilled in the art, under the situation that several improvements and modifications made to the present invention without deviating from the principle of the present invention, the technical solutions obtained by these improvements and modifications also fall within the scope of protection of the claims of the present invention.

## Claims

1. A crystal of the compound shown in formula ①as follows:

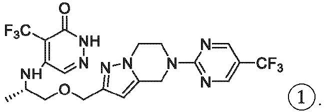

2. The crystal according to claim 1, wherein the X-ray powder diffraction pattern of crystalline form A has characteristic diffraction peaks at the following 2θ positions: 10.389°±0.3°, 11.917°±0.3°, 12.912°±0.3°, 13.385°±0.3°, 14.054°±0.3°, 15.316°±0.3° and 16.636°±0.3°.

3. The crystal according to claim 1, wherein the X-ray powder diffraction pattern of crystalline form A has characteristic diffraction peaks at the following 2θ positions: 6.716°±0.3°, 10.043°±0.3°, 10.389°±0.3°, 11.917°±0.3°, 12.912°±0.3°, 13.385°±0.3°, 14.054°±0.3°, 15.316°±0.3°, 16.636°±0.3°, 18.003°±0.3°, 20.014°±0.3°, 20.794°±0.3° and 23.855°±0.3°.

4. The crystal according to claim 2 or 3, wherein the X-ray powder diffraction pattern of crystalline form A is substantially as shown in FIG. 1.

5. The crystal according to any one of claims 2 to 4, wherein the TGA curve of crystalline form A is substantially as shown in FIG. 10.

6. The crystal according to any one of claims 2 to 4, wherein the DSC curve of crystalline form A is substantially as shown in FIG. 11.

7. A method for preparing crystalline form A according to any one of claims 2 to 6, wherein the compound represented by formula ① is crystallized in solvent (①-A) to obtain crystalline form A, and wherein the solvent (①-A) is selected from any one of acetonitrile, ethanol, n-propanol, acetone and water, or the a mixed solvent of any combination of acetonitrile, ethanol, n-propanol, acetone and water in any ratio.

8. The preparation method according to claim 7, wherein the compound represented by formula ① or the crude product thereof is added to solvent (①-A-1), then the obtained system was heated to for dissolution, then solvent (①-A-2) and solvent (①-A-3) are added, then the obtained system was heated for dissolution, then cooled and stirred continuously, and then allowed to stand for crystallization to obtain crystalline form A, wherein the solvent (①-A-1), solvent (①-A-2), and solvent (①-A-3) are selected from any one of acetonitrile, ethanol, n-propanol, acetone and water.

9. The crystal according to claim 1, wherein the X-ray powder diffraction pattern of crystalline form B has characteristic diffraction peaks at the following 2θ positions: 6.498°±0.3°, 13.326°±0.3°, 21.229°±0.3°, 21.426°±0.3° and 22.195°±0.3°.

10. The crystal according to claim 1, wherein the the X-ray powder diffraction pattern of crystalline form B has characteristic diffraction peaks at the following 2θ positions: 6.498°±0.3°, 9.657°±0.3°, 11.420°±0.3°, 13.027°±0.3°, 13.326°±0.3°, 15.882°±0.3°, 19.075°±0.3°, 19.315°±0.3°, 20.669°±0.3°, 21.229°±0.3°, 21.426°±0.3°, 22.195°±0.3°, 23.118°±0.3° and 23.362°±0.3°.

11. The crystal according to claim 9 or 10, wherein the X-ray powder diffraction pattern of crystalline form B is substantially as shown in FIG. 2.

12. A method for preparing crystalline form B according to any one of claims 9 to 11, wherein the compound represented by formula ① is slurried in solvent (①-B) to obtain crystalline form B, and wherein the solvent (①-B) is selected from n-hexane.

13. The crystal according to claim 1, wherein the X-ray powder diffraction pattern of crystalline form C has characteristic diffraction peaks at the following 2θ positions: 19.066°±0.3°, 20.300°±0.3°, 20.978°±0.3° and 21.616°±0.3°.

14. The crystal according to claim 1, wherein the X-ray powder diffraction pattern of crystalline form C has characteristic diffraction peaks at following 2θ positions: 6.790°±0.3°, 9.917°±0.3°, 11.533°±0.3°, 12.600°±0.3°, 13.556°±0.3°, 13.793°±0.3°, 14.275°±0.3°, 15.008°±0.3°, 16.452°±0.3°, 17.877°±0.3°, 19.066°±0.3°, 19.774°±0.3°, 20.300°±0.3°, 20.978°±0.3°, 21.616°±0.3°, 22.585°±0.3° and 23.407°±0.3°.

15. The crystal according to claim 13 or 14, wherein the X-ray powder diffraction pattern of crystalline form C is substantially as shown in FIG. 3.

16. A method for preparing crystalline form C according to any one of claims 13 to 15, wherein the compound represented by formula ① is crystallized in solvent (①-C) to obtain crystalline form C, and wherein the solvent (①-C) is selected from isopropyl acetate, n-hexane or a mixed solvent of isopropyl acetate and n-hexane.

17. The crystal according to claim 1, wherein X-ray powder diffraction pattern of crystalline form D has characteristic diffraction peaks at the following 2θ positions: 6.646°±0.3° and 13.175°±0.3°.

18. The crystal according to claim 1, wherein the X-ray powder diffraction pattern of crystalline form D has characteristic diffraction peaks at the following 2θ positions: 6.646°±0.3°, 13.175°±0.3°, 13.489°±0.3°, 16.450°±0.3°, 20.950°±0.3°, 21.585°±0.3°, 22.369°±0.3°, 23.036°±0.3° and 23.281°±0.3°.

19. The crystal according to claim 17 or 18, wherein the X-ray powder diffraction pattern of crystalline form D is substantially as shown in FIG. 4.

20. A method for preparing crystalline form D according to any one of claims 17 to 19, wherein the compound represented by formula ① is extracted with solvent (①-D), concentrated and dried under vacuum to obtain crystalline form D, and wherein the solvent (①-D) is selected from ethyl acetate.

21. The crystal according to claim 1, wherein the X-ray powder diffraction pattern of crystalline form E has characteristic diffraction peaks at the following 2θ positions: 18.227°±0.3°, 19.954°±0.3° and 22.449°±0.3°.

22. The crystal according to claim 1, wherein the X-ray powder diffraction pattern of crystalline form E has characteristic diffraction peaks at the following 2θ positions: 8.129°±0.3°, 11.118°±0.3°, 11.360°±0.3°, 11.749°±0.3°, 13.482°±0.3°, 14.730°±0.3°, 18.227°±0.3°, 19.954°±0.3°, 20.712°±0.3°, 21.092°±0.3°, 22.449°±0.3°, 22.813°±0.3°, 24.399°±0.3°, 24.560°±0.3°, 25.923°±0.3°, 26.429°±0.3°, 27.060°±0.3° and 27.446°±0.3°.

23. The crystal according to claim 21 or 22, wherein the X-ray powder diffraction pattern of crystalline form E is substantially as shown in FIG. 5.

24. A method for preparing crystalline form E according to any one of claims 21 to 23, wherein the compound represented by formula ① is crystallized in solvent (①-E) to obtain crystalline form E, and wherein the solvent (①-E) is selected from ethyl acetate, n-hexane or a mixed solvent of ethyl acetate and n-hexane.

25. The crystal according to claim 1, wherein the X-ray powder diffraction pattern of crystalline form F has characteristic diffraction peaks at the following 2θ positions: 6.717°±0.3° and 13.484°±0.3°.

26. The crystal according to claim 1, wherein the X-ray powder diffraction pattern of crystalline form F has characteristic diffraction peaks at the following 2θ positions: 6.717°±0.3°, 9.812°±0.3°, 11.426°±0.3°, 12.501°±0.3°, 13.484°±0.3°, 14.915°±0.3°, 16.374°±0.3°, 16.884°±0.3°, 17.828°±0.3°, 18.975°±0.3°, 20.241°±0.3°, 20.902°±0.3°, 21.593°±0.3°, 22.506°±0.3°, 22.918°±0.3° and 23.412°±0.3°.

27. The crystal according to claim 25 or 26, wherein the X-ray powder diffraction pattern of crystalline form F is substantially as shown in FIG. 6.

28. The crystal according to any one of claims 25 to 27, wherein the TGA curve of crystalline form F is substantially as shown in FIG. 12.

29. The crystal according to any one of claims 25 to 27, wherein the DSC curve of crystalline form F is substantially as shown in FIG. 13.

30. A method for preparing crystalline form F according to any one of claims 25 to 29, wherein the compound represented by formula ① is crystallized in solvent (①-F) to obtain crystalline form F, and wherein the solvent (①-F) is selected from n-propanol, n-heptane or a mixed solvent of n-propanol and n-heptane.

31. The crystal according to claim 1, wherein the X-ray powder diffraction pattern of crystalline form G has characteristic diffraction peaks at the following 2θ positions: 6.743°±0.3° and 13.503°±0.3°.

32. The crystal according to claim 1, wherein the X-ray powder diffraction pattern of crystalline form G has characteristic diffraction peaks at the following 2θ positions: 6.743°±0.3°, 13.503°±0.3°, 16.901°±0.3°, 18.996°±0.3°, 20.260°±0.3°, 20.920°±0.3° and 21.604±0.3°.

33. The crystal according to claim 31 or 32, wherein the X-ray powder diffraction pattern of crystalline form G is substantially as shown in FIG. 7.

34. A method for preparing crystalline form G according to any one of claims 31 to 33, wherein the compound represented by formula ① is crystallized in solvent (①-G) to obtain crystalline form G, and wherein the solvent (①-G) is selected from ethanol, *n*-heptane or a mixed solvent of ethanol and *n*-heptane.

35. The crystal according to claim 1, wherein X-ray powder diffraction pattern of crystalline form H has characteristic diffraction peaks at the following 2θ positions: 12.366°±0.3°, 13.115°±0.3°, 14.359°±0.3°, 15.617°±0.3° and 16.909°±0.3°.

36. The crystal according to claim 1, wherein diffraction pattern of X-ray powder of crystalline form H has characteristic diffraction peaks at the following 2θ positions: 6.514°±0.3°, 10.074°±0.3°, 10.684°±0.3°, 12.366°±0.3°, 13.115°±0.3°, 14.359°±0.3°, 15.617°±0.3°, 16.909°±0.3°, 19.839°±0.3°, 20.092°±0.3°, 20.687°±0.3° and 22.919°±0.3°.

37. The crystal according to claim 35 or 36, wherein the X-ray powder diffraction pattern of crystalline form H is substantially as shown in FIG. 8.

38. The crystal according to any one of claims 35 to 37, wherein the TGA curve of crystalline form H is substantially as shown in FIG. 14.

39. The crystal according to any one of claims 35 to 37, wherein the DSC curve of crystalline form H is substantially as shown in FIG. 15.

40. A method for preparing crystalline form H according to any one of claims 35 to 39, wherein the compound represented by formula ① is crystallized in solvent (①-H) to obtain crystalline form H, and wherein the solvent (①-H) is selected from any one of acetonitrile, ethanol, *n-*propanol, acetone and water, or a mixed solvent of any combinations of acetonitrile, ethanol, *n-*propanol, acetone and water in any ratio.

41. The crystal according to claim 1, wherein the X-ray powder diffraction pattern of crystalline form I has characteristic diffraction peaks at the following 2θ positions: 4.968°±0.3°, 19.770°±0.3° and 21.752°±0.3°.

42. The crystal according to claim 1, wherein the X-ray powder diffraction pattern of crystalline form I has characteristic diffraction peaks at the following 2θ positions: 4.968°±0.3°, 8.863°±0.3°, 12.739°±0.3°, 13.896°±0.3°, 14.848°±0.3°, 18.840°±0.3°, 19.179°±0.3°, 19.770°±0.3°, 21.155°±0.3°, 21.752°±0.3° and 24.053°±0.3°.

43. The crystal according to claim 41 or 42, wherein of the X-ray powder diffraction pattern of crystalline form I is substantially as shown in FIG. 8.

44. A method for preparing crystalline form I according to any one of claims 41 to 43, wherein the compound represented by formula ① is crystallized in solvent (①-I) to obtain crystalline form I, and wherein the solvent (①-I) is selected from acetonitrile, water or a mixed solvent of acetonitrile and water.

45. A pharmaceutical composition comprising a therapeutically effective amount of the crystal according to any one of the claims 1 to 44, and a pharmaceutically acceptable carrier or excipient.

46. Use of the crystal according to any one of claims 1 to 44, or the pharmaceutical composition according to claim 45 in the preparation of medicaments for treating and/or preventing cancers.
